# EUROPEAN PATENT SPECIFICATION

(11) **EP 2 540 839 B1**
(45) Date of publication and mention of the grant of the patent: **01.07.2015**
(21) Application number: 11747526.9
(22) Date of filing: 25.02.2011
(51) Int. Cl.: C12Q 1/68, C12N 15/09, G01N 33/50, G01N 33/53

(54) **METHOD FOR DETERMINING PRESENCE OR ABSENCE OF CANCER CELL IN BIOLOGICAL SAMPLE, AND MOLECULAR MARKER AND KIT FOR DETERMINATION**
VERFAHREN ZUR BESTIMMUNG DER PRÄSENZ ODER ABSENZ VON KREBSZELLEN IN EINER BIOLOGISCHEN PROBE SOWIE MOLEKULARER MARKER UND KIT DAFÜR
PROCÉDÉ POUR DÉTERMINER LA PRÉSENCE OU L'ABSENCE DE CELLULES CANCÉREUSES DANS UN ÉCHANTILLON BIOLOGIQUE, ET MARQUEUR MOLÉCULAIRE ET KIT POUR DÉTERMINATION

(30) Priority: 26.02.2010 JP 2010042814
(43) Date of publication of application: 02.01.2013
(73) Proprietor: Sysmex Corporation, Kobe-shi, Hyogo 651-0073 (JP)
(72) Inventor: SAKAI, Ayako, Kobe-shi Hyogo 651-0073 (JP); TASHIMA, Yoshihiko, Kobe-shi Hyogo 651-0073 (JP); KAJITA, Masahiro, Kobe-shi Hyogo 651-0073 (JP)
(74) Representative: Paemen, Liesbet R.J.
(86) International application number: PCT/JP2011/054363
(87) International publication number: WO 2011/105570

(56) References cited:
- WO-A1-03/074736
- MARTIN WIDSCHWENDTER ET AL: "HOXA methylation in normal endometrium from premenopausal women is associated with the presence of ovarian cancer: A proof of principle study", INTERNATIONAL JOURNAL OF CANCER, vol. 125, no. 9, 1 November 2009 (2009-11-01), pages 2214-2218, XP055066843, ISSN: 0020-7136, DOI: 10.1002/ijc.24599
- GE LIND ET AL: "Novel epigenetically deregulated genes in testicular cancer include homeobox genes and SCGB3A1 ( HIN-1 )", THE JOURNAL OF PATHOLOGY, vol. 210, no. 4, 1 December 2006 (2006-12-01), pages 441-449, XP055066840, ISSN: 0022-3417, DOI: 10.1002/path.2064
- WU Q. ET AL.: 'DNA methylation profiling of ovarian carcinomas and their in vitro models identifies HOXA9, HOXB5, SCGB3A1, and CRABP1 as novel targets.' MOLECULAR CANCER vol. 6, no. 45, 2007, pages 1 - 10
- RAUCH T. ET AL: 'Homeobox gene methylation in lung cancer studied by genome-wide analysis with a microarray-based methylated CpG island recovery assay' PROCEEDINGS OF THE NATIONAL ACADEMY OF SCIENCES OF USA vol. 104, no. 13, 2007, pages 5527 - 5532, XP002577090
- RUIKE Y. ET AL: 'Genome-wide analysis of aberrant methylation in human breast cancer cells using methyl-DNA immunoprecipitation combined with high-throughput sequencing' BMC GENOMICS vol. 11, no. 137, 25 February 2010, pages 1 - 11
- JACINTO F.V. ET AL: 'Methyl-DNA immunoprecipitation (MeDIP): Hunting down the DNA methylome' BIOTECHNIQUES vol. 44, no. 1, 2008, page 35, 37, 39, 41, 43, XP001525871
- YOSHIHIKO TAJIMA ET AL.: 'Shinki Kaiseki Shuho o Mochiita MeDIP on chip assay no Yuyosei Hyoka to, Nyugan ni Okeru Shinki Methyl-ka Idenshi Marker Kensaku, #P-0383' DAI 68 KAI PROCEEDINGS OF THE JAPANESE CANCER ASSOCIATION vol. 68, 2009, page 179

## Description

### TECHNICAL FIELD

The present invention relates to a method for determination of presence or absence of a cancer cell in a biological sample. More specifically, the present invention relates to the method for determining presence or absence of a cancer cell in a biological sample based on an analysis result obtained by analyzing methylation status of a CpG site in a certain base sequence for a DNA extracted from the biological sample.

The present invention also relates to a molecular marker and a kit for determination which are used for the above method.

### BACKGROUND ART

It has been known that chromosome DNAs of higher eukaryotes may undergo methylation at the 5-position of C (cytosine) among other bases constituting DNAs. Such DNA methylation functions as a mechanism for suppression of gene expression. For example, when a region rich in CpG sequences (also referred to as "CpG islands"), which often exists in promoter regions of certain genes, is methylated, transcription of these genes may be suppressed. This phenomenon is referred to as "gene silencing". On the other hand, when a CpG island is not methylated, a transcription factor can bind to the promoter region and the gene can be transcribed.

Accordingly, DNA methylation is one of control mechanisms of gene expression. For this reason, DNA methylation plays important roles in various physiological and pathological phenomena such as early embryonic development, expression of tissue specific genes, genomic imprinting and X chromosome inactivation which are characteristic to mammals, stabilization of chromosomes, synchronization of DNA replication and the like.

Recently, it has also been revealed that abnormal DNA methylation, i.e. gene silencing due to DNA methylation is involved in development and progress of diseases such as cancers. Accordingly, it has been attempted to diagnose diseases such as cancers by analyzing methylation status of DNA for various genes. EP1489189 (WO03/074736) discloses genes whose methylation status is altered in cancer cells. Patent Literature 1, for example, discloses that genes such as NPTX2, SARP2 and CLDN5 and the like are not methylated in normal pancreatic cells while they are methylated in pancreatic cancer cells, and describes a method for detection of pancreatic cancer by analyzing methylation status of these genes. Patent Literature 2 discloses a method for detection of one type of breast cancers, proliferative breast disease, by analyzing methylation status of genes such as BRCA2 and PCDH7 and the like. Rauch et al (Proc. Natl. Acad. Sci. 104(13):5527-5532, 2007) discloses a difference in the overall methylation status of the HOXA9 gene between different types of testicular germ cell tumors (TGCTs).

A difference in the methylation status of the HoXAq gene in ovarian and lung cancer is respectively also discloses in:
MARTIN WIDSCHWENDTER ET AL: "HOXA methylation in normal endometrium from premenopausal women is associated with the presence of ovarian cancer: A proof of principle study", INTERNATIONAL JOURNAL OF CANCER, vol. 125, no. 9, 1 November 2009 (2009-11-01), pages 2214-2218.
GE LIND ET AL: "Novel epigenetically deregulated genes in testicular cancer include homeobox genes and SCGB3A1 ( HIN-1 )", THE JOURNAL OF PATHOLOGY, vol. 210, no. 4, 1 December 2006 (2006-12-01), pages 441-449,

As described above, many genes have been reported which are abnormally methylated in various cancers. However, only few among them can be used as molecular markers for detection of cancers.

Accordingly, there is a need for further development of novel molecular markers useful to be used in a detection method of a cancer cell utilizing gene methylation analysis.

### Citation List

### Patent Literature

Patent Literature 1: Japanese Translation of PCT International Application No. 2007-524369 (corresponding to PCT International Publication WO 2004/083399)

Patent Literature 2: Japanese Translation of PCT International Application No. 2008-506407 (corresponding to PCT International Publication WO 2006/008128)

### SUMMARY OF INVENTION

### Technical Problem

An object of the present invention is to provide a method for determination of presence or absence of a cancer cell based on an analysis result obtained by analyzing methylation status of DNA extracted from a biological sample with novel molecular markers allowing determination of presence or absence of a cancer cell according to claim 1.

Another object of the present invention is to provide and a kit for determination which can be used in the above method.

### Solution to Problem

The present inventors analyzed methylation status of genomic DNA from cancer tissues and cancer cell lines and identified gene regions in which DNA methylation was found specifically for cancer tissues and cancer cell lines. The present inventors further found that the analysis result obtained by analyzing methylation status for certain base sequences in these gene regions allows highly accurate differentiation between samples containing a cancer cell and the ones without a cancer cell, thereby completing the present invention.

Namely, the present invention provides a method for determination of presence or absence of a cancer cell in a biological sample obtained from a subject comprising the steps of:
extracting DNA from the biological sample;
analyzing methylation status, for the DNA obtained in the extracting step,by performing methylation-specific PCR using a primer set amplifying at least one CpG site chosen from the 9th, 10th and 28th to 30th CpG sites from the 5' end of a base sequence of SEQ ID NO: 1 of the HOXA9 gene; and
determining presence or absence of the cancer cell in the biological sample based on a result of of the PCR, wherein it is determined that the cancer cell is present in the biological sample when amplified product of the PCR is detected.

The present invention also provides the in vitro use of a primer set amplifying at least one CpG site chosen from the 9th, 10th and 28th to 30th CpG sites from the 5' end of a base sequence of SEQ ID NO: 1 of the HOXA9 gene for determination of presence of absence of a cancer cell in a biological sample obtained from a subject by methylation specific PCR.

The present invention further provides a kit for determination of presence or absence of a cancer cell in a biological sample obtained from a subject comprising:
a non-methylated cytosine conversion agent that converts non-methylated cytosine in DNA extracted from the biological sample to a different base; and
a primer set for amplifying at least one CpG site chosen from the 9th, 10th and 28th to 30th CpG sites from the 5' end of a base sequence of SEQ ID NO: 1 of the HOXA9 gene by methylation specific PCR.

### Advantageous Effects of Invention

According to the present method for determination of presence or absence of a cancer cell in a biological sample (hereinafter also referred to as "the present method"), presence or absence of a cancer cell in a biological sample obtained from a subject can be determined by analyzing methylation status of the present molecular marker(s) for DNA extracted from the biological sample.

The present invention can further provide a kit which can be used for the method as disclosed in claim 1.

### BRIEF DESCRIPTION OF DRAWINGS

Fig. 1 is a table showing methylation status of the 1st to 54th CpG sites from the 5' end of the base sequence SEQ ID NO: 1 in DNA obtained from 4 pancreatic cancer tissue samples and 5 normal pancreatic tissue samples;
Fig. 2 is a table showing methylation status of the 1st to 79th CpG sites from 5' end of the base sequence SEQ ID NO: 2 in DNA obtained from 5 pancreatic cancer tissue samples and 5 normal pancreatic tissue samples;
Fig. 3 is a table showing methylation status of the 1st to 12th CpG sites from 5' end of the base sequence SEQ ID NO: 3 in DNA obtained from 5 pancreatic cancer tissue samples and 4 normal pancreatic tissue samples;
Fig. 4 is a bar graph showing methylation frequency of CpG sites located in the respective base sequences SEQ ID NOs: 1 to 3;
Fig. 5 is a bar graph showing fluorescence intensity of the bands of PCR products obtained by amplifying the base sequence SEQ ID NO: 15 in DNA obtained from pancreatic cancer tissue and normal tissue by a methylation specific PCR (MSP) method;
Fig. 6 is a bar graph showing fluorescence intensity of the bands of PCR products obtained by amplifying the base sequence SEQ ID NO: 16 in DNA obtained from pancreatic cancer tissue and normal tissue by the MSP method;
Fig. 7 is a bar graph showing fluorescence intensity of the bands of PCR products obtained by amplifying the base sequence SEQ ID NO: 17 in DNA obtained from pancreatic cancer tissue and normal tissue by the MSP method;
Fig. 8 is a bar graph showing fluorescence intensity of the bands of PCR products obtained by amplifying the base sequence SEQ ID NO: 18 in DNA obtained from pancreatic cancer tissue and normal tissue by the MSP method;
Fig. 9 is a bar graph showing fluorescence intensity of the bands of PCR products obtained by amplifying the base sequence SEQ ID NO: 19 in DNA obtained from pancreatic cancer tissue and normal tissue by the MSP method;
Fig. 10 is a bar graph showing fluorescence intensity of the bands of PCR products obtained by amplifying the base sequence SEQ ID NO: 15 in DNA obtained from large intestinal tissue and mammary gland tissue by the MSP method;
Fig. 11 is a bar graph showing fluorescence intensity of the bands of PCR products obtained by amplifying the base sequence SEQ ID NO: 18 in DNA obtained from mammary gland tissue by the MSP method;
Fig. 12 is a table showing methylation status of the 1st to 52nd CpG sites from the 5' end of the base sequence SEQ ID NO: 7 in 6 samples of a breast cancer cell line MCF7 and 5 samples of a normal mammary gland epithelial cell line HMEC;
Fig. 13 is a table showing methylation status of the 1st to 39th CpG sites from the 5' end of the base sequence SEQ ID NO: 8 in 4 MCF7 cell samples and 4 HMEC cell samples;
Fig. 14 is a table showing methylation status of the 1st to 23rd CpG sites from the 5' end of the base sequence SEQ ID NO: 9 in 6 MCF7 cell samples and 6 HMEC cell samples;
Fig. 15 is a table showing methylation status of the 1st to 10th CpG sites from the 5' end of the base sequence SEQ ID NO: 10 in 5 MCF7 cell samples and 5 HMEC cell samples;
Fig. 16 is a table showing methylation status of the 1st to 23rd CpG sites from the 5' end of the base sequence SEQ ID NO: 11 in 4 MCF7 cell samples and 4 HMEC cell samples;
Fig. 17 is a table showing methylation status of the 1st to 32nd CpG sites from the 5' end of the base sequence SEQ ID NO: 12 in 6 MCF7 cell samples and 6 HMEC cell samples;
Fig. 18 is a table showing methylation status of the 1st to 19th CpG sites from the 5' end of the base sequence SEQ ID NO: 13 in 5 MCF7 cell samples and 4 HMEC cell samples;
Fig. 19 is a table showing methylation status of the 1st to 24th CpG sites from the 5' end of the base sequence SEQ ID NO: 14 in 4 MCF7 cell samples and 4 HMEC cell samples;
Fig. 20 a bar graph showing methylation frequency of CpG sites located in the respective base sequences SEQ ID NOs: 7 to 14;
Fig. 21 is a bar graph showing fluorescence intensity of the bands of PCR products obtained by amplifying the base sequence SEQ ID NO: 20 in DNA obtained from MCF7 cells and HMEC cells by the MSP method;
Fig. 22 is a bar graph showing fluorescence intensity of the bands of PCR products obtained by amplifying the base sequence SEQ ID NO: 21 in DNA obtained from MCF7 cells and HMEC cells by the MSP method;
Fig. 23 is a bar graph showing fluorescence intensity of the bands of PCR products obtained by amplifying the base sequence SEQ ID NO: 22 in DNA obtained from MCF7 cells and HMEC cells by the MSP method;
Fig. 24 is a bar graph showing fluorescence intensity of the bands of PCR products obtained by amplifying the base sequence SEQ ID NO: 23 in DNA obtained from MCF7 cells and HMEC cells by the MSP method;
Fig. 25 is a bar graph showing fluorescence intensity of the bands of PCR products obtained by amplifying the base sequence SEQ ID NO: 24 in DNA obtained from MCF7 cells and HMEC cells by the MSP method;
Fig. 26 is a bar graph showing fluorescence intensity of the bands of PCR products obtained by amplifying the base sequence SEQ ID NO: 25 in DNA obtained from MCF7 cells and HMEC cells by the MSP method;
Fig. 27 is a bar graph showing fluorescence intensity of the bands of PCR products obtained by amplifying the base sequence SEQ ID NO: 26 in DNA obtained from MCF7 cells and HMEC cells by the MSP method;
Fig. 28 is a bar graph showing fluorescence intensity of the bands of PCR products obtained by amplifying the base sequence SEQ ID NO: 27 in DNA obtained from MCF7 cells and HMEC cells by the MSP method;
Fig. 29 is a bar graph showing fluorescence intensity of the bands of PCR products obtained by amplifying the base sequence SEQ ID NO: 20 in DNA obtained from breast cancer tissue and normal mammary gland tissue by the MSP method;
Fig. 30 is a bar graph showing fluorescence intensity of the bands of PCR products obtained by amplifying the base sequence SEQ ID NO: 21 in DNA obtained from breast cancer tissue and normal mammary gland tissue by the MSP method;
Fig. 31 is a bar graph showing fluorescence intensity of the bands of PCR products obtained by amplifying the base sequence SEQ ID NO: 22 in DNA obtained from breast cancer tissue and normal mammary gland tissue by the MSP method;
Fig. 32 is a bar graph showing fluorescence intensity of the bands of PCR products obtained by amplifying the base sequence SEQ ID NO: 23 in DNAs obtained from breast cancer tissue and normal mammary gland tissue by the MSP method;
Fig. 33 is a bar graph showing fluorescence intensity of the bands of PCR products obtained by amplifying the base sequence SEQ ID NO: 24 in DNA obtained from breast cancer tissue and normal mammary gland tissue by the MSP method;
Fig. 34 is a bar graph showing fluorescence intensity of the bands of PCR products obtained by amplifying the base sequence SEQ ID NO: 25 in DNA obtained from breast cancer tissue and normal mammary gland tissue by the MSP method;
Fig. 35 is a bar graph showing fluorescence intensity of the bands of PCR products obtained by amplifying the base sequence SEQ ID NO: 26 in DNA obtained from breast cancer tissue and normal mammary gland tissue by the MSP method;
Fig. 36 is a bar graph showing fluorescence intensity of the bands of PCR products obtained by amplifying the base sequence SEQ ID NO: 27 in DNA obtained from breast cancer tissue and normal mammary gland tissue by the MSP method;
Fig. 37 shows photographs obtained after agarose electrophoresis of reaction solutions resulting from amplification of base sequences SEQ ID NOs: 17 to 19, 21, 22 and 25 to 27 in DNA obtained from colon cancer tissue and normal large intestinal tissue by the MSP method; and
Fig. 38 shows photographs obtained after agarose electrophoresis of reaction solutions resulting from amplification of base sequences SEQ ID NOs: 20 to 27 in DNA obtained from MCF7 cells, normal heart tissue, normal kidney tissue, normal liver tissue, normal lung tissue and normal peripheral blood leukocytes by the MSP method.

### DESCRIPTION OF EMBODIMENTS

As used herein, the term "CpG site" means a site of a sequence in a base sequence in which cytosine (C) and guanine (G) are adjacent in this order from 5' to 3'. The letter "p" in "CpG" represents a phosphodiester bond between cytosine and guanine.

As used herein, to "analyze methylation status" means to analyze presence or absence of methylation of a CpG site located in a base sequence to be analyzed or methylation frequency of a CpG site(s) in the base sequence. In this context, a base sequence to be analyzed is not specifically limited so long as it is a region comprising at least one CpG site chosen from the 9th, 10th and 28th to 30th CpG sites from the 5' end of a base sequence of SEQ ID NO: 1 of the HOXA9 gene.

The term "presence or absence of methylation" means whether or not a cytosine residue in a CpG site located in a base sequence to be analyzed is methylated.

The term "methylation frequency" means a ratio of the number of methylated CpG site(s) relative to the total number of CpG site(s) located in a base sequence to be analyzed. The position and number of CpG site(s) located in base sequence SEQ ID NOs: 1 is known. Accordingly, if the base sequence to be analyzed is SEQ ID NOs: 1, the number of CpG site(s) located therein can be understood beforehand. Therefore, the number of methylated CpG site(s) itself in the base sequence to be analyzed can also be used as the methylation frequency.

According to the present method, DNA is first extracted from a biological sample obtained from a subject.

In this extracting step, the biological sample is not specifically limited so long as it contains DNA of a subject, and is preferably a sample containing genomic DNA, e.g. a clinical specimen. The clinical specimen can specifically include blood, serum, plasma, lymph fluid, urine, nipple discharge, tissue and cells obtained by operations and biopsies and the like.

DNA can be extracted from a biological sample by well-known extraction methods. Extraction of DNA can be carried out, for example, by mixing a biological sample with a treatment solution containing a surfactant for solubilization of cells or tissues (e.g. sodium cholate, sodium dodecyl sulfate etc.), and subjecting the resulting mixture to physical procedure (stirring, homogenization, ultrasonication etc.) to release DNA contained in the biological sample into the mixture. In this case, it is preferable to centrifuge the mixture to precipitate cell debris, collect the supernatant containing DNA and use the supernatant to the next step of analyzing. The obtained supernatant can be purified according to well-known methods. DNA can also be extracted and purified from a biological sample by using commercially available kits.

The extracting step preferably further comprises a step of fragmenting the extracted DNA. According to this step, DNA can be fragmented into appropriate lengths, allowing effective methylated DNA immunoprecipitation (MeDIP) and non-methylated cytosine conversion being carried out.

Fragmentation of DNA can be carried out by ultrasonication, alkaline treatment, restriction enzyme treatment and the like. When the alkaline treatment is carried out with sodium hydroxide, sodium hydroxide is added to a DNA solution to the final concentration of 0.1 to 1.0N and the mixture is incubated at 10 to 40°C for 5 to 15 minutes to fragment the DNA. In case of the restriction enzyme treatment, the restriction enzyme can appropriately be selected based on the base sequence of DNA, which can be *Mse*I or *Bam*HI, for example.

Next, according to the present method, methylation status of at least one CpG site chosen from the 9th, 10th and 28th to 30th CpG sites located in the 5' end of a base sequence of SEQ ID NO: 1 of the HOXA9 gene is analyzed for the DNA extracted from the biological sample.

SEQ ID NOs: 1 is a partial region of human genomic DNA. More specifically, this base sequence is a part of a gene region described in Table 1 or a base sequence region in the vicinity thereof. The base sequence per se is well-known and can be obtained from well-known databases such as the one provided by the National Center for Biotechnology Information (NCBI) (http://www.ncbi.nlm.nih.gov/gene/).

**[Table 1]**

| **SEQ ID NO:** | **Entrez gene ID** | **Gene symbol** | **Gene title** |
|---|---|---|---|
| 1 | 3205 | HOXA9 | Homeobox A9 |
| 2 | 55539 | KCNQ1DN | KCNQ1 downstream neighbor |
| 3 | 5913 | RAPSN | Receptor-associated protein of the synapse |
| 4 and 5 | 8326 | FZD9 | Frizzled homolog 9 |
| 6 | 64405 | CDH22 | Cadherin 22 |
| 7 | 85474 | LBX2 | Ladybird homeobox 2 |
| 8 | 5083 | PAX9 | Paired box 9 |
| 9 | 120 | ADD3 | Adducin 3 |
| 10 | 232933 | CCDC61 | Coiled coli domain containing 61 |
| 11 | 84107 | ZIC4 | Zic family member 4 |
| 12 | 94027 | CGB7 | Chorionic gonadotropin beta polypeptide 7 |
| 13 | 9760 | TOX | Thymocyte selection associated high mobility group box |
| 14 | 7137 | TNNI3 | Troponin I type 3 |

In the analyzing step envisaged herein, the CpG site to be analyzed is preferably selected from the 9th, 10th and 28th to 30th CpG sites from the 5' end of the base sequence SEQ ID NO: 1;

In this case, one CpG site can be analyzed; however, in order to improve determination accuracy in the subsequent determining step, more than one CpG site is preferably analyzed for presence or absence of methylation. More than one CpG site can be selected from SEQ ID NO:1.

Alternatively, the analyzing step can be a step of analyzing methylation frequency of a CpG site(s) selected from one or more of the 9th, 10th and 28th to 30th CpG sites located in the 5' end of SEQ ID NO:1.

In this case, the base sequence to be analyzed can contain only one CpG site; however, in order to improve determination accuracy in the subsequent determining step, it is preferable that the base sequence to be analyzed contains more than one CpG site. The base sequence to be analyzed is at least SEQ ID NOs: 1; however, more than one base sequence among them is preferably analyzed.

Various methods are well-known as the method for analyzing methylation status. According to the present method, it is not specifically limited as to which analysis method is used; however, the analysis method preferably comprises differentiating between methylated DNA and non-methylated DNA, amplifying DNA and detecting methylated DNA and/or non-methylated DNA.

The step of differentiation between methylated DNA and non-methylated DNA can include a step of carrying out methylation sensitive restriction enzyme treatment, a MeDIP method, non-methylated cytosine converting treatment and the like.

The step of amplifying DNA can include a step of carrying out PCR amplification, quantitative PCR amplification, IVT (*in vitro* transcription) amplification, SPIA™ amplification and the like methods.

The step of detecting methylated DNA and/or non-methylated DNA can include a step of carrying out electrophoresis, sequence analysis, microarray analysis, mass spectrometry, Southern hybridization and the like.

The MeDIP method is the method in which methylated DNA in a biological sample is concentrated by immunoprecipitation using an anti-methylated cytosine antibody or an anti-methylated cytidine antibody, or an antibody which specifically recognizes a methylated DNA-binding protein. According to the analyzing step of the present method, methylated DNA comprised in DNA obtained in the extracting step can be concentrated by the MeDIP method and methylation status of the concentrated methylated DNA can be analyzed.

The methylated DNA concentrated by the MeDIP method can be amplified by e.g. IVT amplification and methylation status of the obtained amplified products can be analyzed by using a microarray. These analysis procedures are referred to as the MeDIP on chip method.

The non-methylated cytosine converting treatment is the one in which DNA extracted from a biological sample is subjected to a reaction with a non-methylated cytosine conversion agent so as to convert non-methylated cytosine(s) in the DNA to a different base (uracil, thymine, adenine or guanine).

In this context, the non-methylated cytosine conversion agent is a substance which can react with DNA and convert non-methylated cytosine in the DNA to a different base (uracil, thymine, adenine or guanine). The non-methylated cytosine conversion agent suitably used can be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status in DNA is converted to the difference in a base sequence (C and U) by the non-methylated cytosine converting treatment using bisulfite. The non-methylated cytosine converting treatment using bisulfite is referred to as bisulfite treatment.

When the bisulfite treatment is carried out, the amount added of bisulfite (concentration) is not specifically limited so long as it can sufficiently convert non-methylated cytosine(s) in DNA, and corresponds to, for example, 1M or higher, preferably 1 to 15M, more preferably 3 to 10M as the final concentration in the sample. The incubation conditions (temperature and time) after the addition of bisulfite to a biological sample can be appropriately selected according to the amount added of bisulfite, and for example, when bisulfite is added at the final concentration of 6M, the incubation is carried out at 50 to 80°C for 10 to 90 minutes.

Methylation status of DNA can be analyzed by sequencing DNA after bisulfite treatment and detecting the difference in base sequence from the original sequence. This process is referred to as a bisulfite sequencing method.

Methylation status of DNA can also be analyzed by amplifying DNA after bisulfite treatment by PCR using a primer set described hereinafter and determining presence or absence of a PCR product. This analysis procedure is referred to as a methylation specific PCR (MSP) method.

The MSP method utilizes a primer set which can amplify a base sequence in which cytosine in a CpG site to be analyzed is methylated (i.e. cytosine is not converted to uracil) while which can not amplify a base sequence in which cytosine in a CpG site is not methylated (i.e. cytosine is converted to uracil). According to the MSP method using such a primer set, presence of the PCR product indicates methylation of the CpG site analyzed.

The MSP method can also be carried out by using a primer set which can not amplify a base sequence in which cytosine in a CpG site to be analyzed is not converted to uracil, while which can amplify a base sequence in which cytosine in a CpG site is converted to uracil. In this case, absence of the PCR product indicates methylation of the CpG site analyzed.

Each primer in the above primer sets can be appropriately designed by a person skilled in the art based on the base sequence comprising a CpG site to be analyzed, and it is preferably designed so as to contain cytosine of the CpG site to be analyzed at the 3' end or in the vicinity thereof of the primer.

Methylation status can be analyzed with a microarray in the analyzing step of the present method. In this case, the microarray for analysis can be prepared by immobilizing one or more nucleic probes complementary to the base sequence SEQ ID NO: 1 on a substrate. The microarray can be prepared according to well-known methods in the art.

In the analysis using a microarray, DNA extracted from a biological sample is preferably labeled with a labeling substance well-known in the art. Thus, the present method preferably further comprises a step of labeling the extracted DNA. The labeling step is advantageously carried out after the amplifying step because all DNA in the biological sample can be labeled. The labeling substance can include fluorescence substances, haptens such as biotin, radioactive substances and the like. The fluorescence substances can include Cy3, Cy5, Alexa Fluor™, FITC and the like.

Labeling of DNA facilitates measurement of a signal from a probe on a microarray. A method for labeling DNA with the labeling substance is well-known in the art.

The above signal can be any suitable signal according to the type of microarrays. For example, the signal can be an electric signal generated when a DNA fragment hybridizes to a probe on the microarray, or a fluorescence or luminescence signal generated from a labeling substance when DNA to be analyzed is labeled as described above.

Detection of signal can be carried out by using a scanner comprised in a conventional microarray analyzer. The scanner can be, for example, GeneChip^{®} Scanner3000 7G (Affymetrix) and the like.

In the present method, presence or absence of a cancer cell in a biological sample is determined based on the analysis result obtained in the analyzing step.

In the determining step, when the result obtained in the analyzing step indicates that there is a methylated CpG site, it can be determined that a cancer cell is present in a biological sample obtained from a subject. On the other hand, when the result obtained indicates that there is no methylated CpG site, it can be determined that no cancer cell is present in the biological sample.

The determination can be carried out based on the analysis result of one CpG site; however, in order to improve determination accuracy, it is preferable that the determination is made based on the analysis result of more than one CpG site.

In the determining step, when the result obtained in the analyzing step indicates that methylation frequency is higher than a predetermined threshold, it can be determined that a cancer cell is present in a biological sample obtained from a subject. On the other hand, when the result obtained indicates that methylation frequency is lower than a predetermined threshold, it can be determined that no cancer cell is present in a biological sample obtained from a subject.

The above threshold can be predetermined as follows. First, methylation frequency is analyzed for DNA extracted from a biological sample which is confirmed to be devoid of cancer cells (normal tissue or normal cells) and a biological sample containing a cancer cell. Next, based on the obtained analysis results, a threshold is determined within a range which is higher than the methylation frequency of the biological sample devoid of cancer cells and lower than that of the biological sample containing a cancer cell. Preferably, a threshold is determined as a value which can highly accurately differentiate between the biological sample devoid of cancer cells and the biological sample containing a cancer cell.

The present invention also encompasses the in vitro use of a primer set for determination of presence or absence of a cancer cell which can be used for the present method (hereinafter also referred to as "the present molecular marker").

The present primer set is a primer set amplifyingat least one selected from the 9th, 10th and 28th to 30th CpG sites located in the 5' end of base sequence SEQ ID NO: 1.

The present invention also encompasses a kit for determination of presence or absence of a cancer cell in a biological sample for implementing the present method (hereinafter also referred to as "the present kit").

The present kit comprises a non-methylated cytosine conversion agent and a primer set for determination of methylation status of at least one CpG site selected from the 9th, 10th and 28th to 30th CpG sites located in the the 5' end of base sequence SEQ ID NO: 1 by methylation specific PCR.

The non-methylated cytosine conversion agent comprised in the present kit is not specifically limited so long as it can react with DNA extracted from a biological sample obtained from a subject and convert non-methylated cytosine in the DNA to a different base (uracil, thymine, adenine or guanine), and is preferably bisulfite. The non-methylated cytosine conversion agent can be in the form of a solution or in the form of a solid which can be dissolved in an appropriate solvent before use to provide a solution.

When the present kit is used, a CpG site selected from the followings is preferably analyzed by the MSP method:
the 9th, 10th and 28th to 30th CpG sites from the 5' end of the base sequence SEQ ID NO: 1;

The primer set comprised in the present kit is preferably selected from the followings:
a primer set of the base sequences SEQ ID NO: 28 and SEQ ID NO: 29;

The above primer set is suitably used when the present method is carried out by analyzing presence or absence of methylation of a CpG site chosen from the 9th, 10th and 28th to 30th CpG sites from the 5' end of the base sequence SEQ ID NOs: 1 by the MSP method.

The present invention is hereinafter illustrated in further detail by means of examples, which do not limit the present invention. At the same time, not all of the examples fall within the scope of the present claims.

### Examples

### Example 1: Identification of novel markers from genomic DNA of pancreatic cancer tissue

Genomic DNA from human pancreatic cancer tissue and human normal pancreatic tissue was analyzed by a microarray to search gene regions which is specifically methylated in the genomic DNA from pancreatic cancer tissue.

Specific operation procedures carried out in Example 1 followed the instructions attached to the kits and reagents used.

### 1. Preparation of methylated DNA by MeDIP method

Human pancreatic cancer tissue and human normal pancreatic tissue were used as biological samples from which genomic DNA was extracted. The extracted genomic DNA (4 µg) was incubated with the restriction enzyme *Mse*I (NEB Inc.) at 37°C overnight to fragment the DNA into the sizes from 300 to 1000 bp. The reagents were then denatured by heating at 95°C for 10 minutes followed by quenching to 4°C to obtain single-stranded DNA fragments.

The obtained single-stranded DNA fragments were diluted in a dilution buffer (302 µl) included in the Chromatin Immunoprecipitation assay kit (Upstate Biotechnology). The diluted solutions were then added with Protein G Sepharose beads (68 µl: GE Healthcare), rotated at 4°C for an hour and centrifuged to remove proteins and the like which non-specifically bound to the beads (this procedure is hereinafter also called as "pre-clear treatment"). The supernatants were collected and then respectively divided into two aliquots in separate tubes, one of which was added with an anti-methylated cytosine antibody BI-MECY-0500 (10 µg: Eurogentec) and the other with no antibody, serving as a test sample and a control sample (Input), respectively. The respective test samples were rotated overnight at 4°C prior to addition of Protein G Sepharose beads (68 µl: GE Healthcare) and rotation at 4°C for an additional hour. The samples were then centrifuged, the supernatants were removed and the beads were collected. The obtained beads were washed with a washing buffer included in the above kit, and added with an elution buffer (250 µl) to elute single-stranded DNA fragments.

The obtained DNA solutions were incubated with Proteinase K (Sigma) before purification with the Qiaquick PCR purification kit (QIAGEN).

Quantitative PCR allowed confirming that methylated DNA in respective test samples was specifically concentrated.

### 2. Amplification and labeling of nucleic acid in samples

For the test samples and control samples respectively obtained from the human pancreatic cancer tissue and human normal pancreatic tissue, DNA dephosphorylation was carried out with CIP (Calf intestine phosphatase) (New England Biolab). DNA was then added with dATP at its 3' end using Terminal transferase (ROCHE) and purified using the MinElute purification kit (QIAGEN). The single-stranded DNA fragments in the samples were converted to double-stranded DNA by using the GeneChip^{®} One-Cycle Target Labeling kit (Affymetrix). The resulting double-stranded DNA was used as a template and labeled with biotin by IVT amplification to obtain biotinylated cRNA from each sample. The concentration of the obtained cRNA was determined by measuring the absorbance at 260 nm and 280 nm.

The cRNA from each sample was fragmented with the GeneChip^{®} Sample Cleanup Module (Affymetrix) to obtain test and control samples for microarray analysis.

### 3. Microarray analysis

### (1) Contact of sample and microarray

The samples for microarray analysis were brought into contact with the microarray GeneChip^{®} Human Promoter 1.0R Array (Affymetrix) to carry out hybridization with probes. One same type of microarray was used for each of the test sample and the control sample. Staining, washing and scanning (measurement of a signal) after contacting with the microarray were carried out according to the instruction provided by Affymetrix.

### (2) Analysis of microarray data

The resulting microarray data was analyzed according to the following procedures. In this analysis, a conventional computer was used comprising a central processing unit, a memory part, an input part such as a keyboard and an output part such as a display.
(Step 1) The base sequences of all probes on the microarray, the base sequence of the genomic DNA and the signal values measured as above were entered in the computer.
(Step 2) The base sequence of the genomic DNA was sectioned at the recognition sequence "TACC" of the restriction enzyme *Mse*I to obtain the base sequences of the resulting DNA fragments. Among these base sequences, the base sequences of DNA fragments were extracted which have 300 bp or more in length and do not comprise the sequence "CG". Accordingly, the base sequences of DNA fragments of 300 bp or more without the "CG" sequence were obtained (hereinafter also referred to as "fragment sequences").
(Step 3) Among all probes on the microarray, probes which do not contain the "CG" sequence were extracted. Probes which were complementary to the above fragment sequences among the extracted probes were selected as corrective probes.
(Step 4) Signal values obtained with the corrective probes were extracted from the measured signal values entered and their statistically representative value (mode) was calculated. This value was used as the background value. This procedure was carried out respectively for the test samples and the control samples.
(Step 5) The background value was subtracted from the respective measured signal values entered and the obtained values served as corrected values. When a corrected value is negative, the corrected value was regarded as zero. This procedure was also carried out respectively for the test samples and the control samples.
(Step 6) The Wilcoxon's signed rank sum test was carried out for the corrected values of the test and control samples to calculate the significance probability.

The obtained significance probability was visualized with a default browser IGB (Integrated Genome Browser; IGB). The significance probability displayed in IGB is the one which has been converted with the formula: (converted value) = -10 log10(significance probability). When the converted value is 20 or more, it is considered that a gene region containing a CpG site which is specifically methylated in the genomic DNA of pancreatic cancer tissue could be significantly detected.

As a result, the regions represented by the base sequences SEQ ID NOs: 15 to 19 were identified as the gene regions which were specifically methylated in genomic DNA of pancreatic cancer tissue. The base sequences SEQ ID NOs: 15 to 19 are the gene regions or partial base sequence regions in the vicinity thereof of, respectively, *HOXA9, KCNQ1DN, RAPSN, FZD9* and *CDH22* and the regions comprising the base sequences SEQ ID NOs: 1, 2, 3, 4 and 5 as well as 6, respectively.

The correspondence relationship between the base sequences SEQ ID NOs: 15 to 19 and SEQ ID NOs: 1 to 6 and the above genes is summarized in the following Table 2.

**[Table 2]**

| SEQ ID NO: | Corresponding SEQ ID NO: | Gene |
|---|---|---|
| 15 | 1 | HOXA9 |
| 16 | 2 | KCNQ1DN |
| 17 | 3 | RAPSN |
| 18 | 4 and 5 | FZD9 |
| 19 | 6 | CDH22 |

### Example 2: Analysis of methylation status by bisulfite sequencing

By using bisulfite sequencing, it was investigated whether CpG sites in the gene regions represented by the base sequences SEQ ID NOs: 15 to 17 identified in Example 1 were methylated in genomic DNA from pancreatic cancer tissue and human normal pancreatic tissue.

### 1. Bisulfite treatment

To genomic DNA (2 µg) extracted from human pancreatic cancer tissue and human normal pancreatic tissue used in Example 1 was added 300 µl of a 0.3N sodium hydroxide solution prior to incubation at 37°C for 10 minutes in order to denature the genomic DNA. To each DNA solution was added 300 µl of a 10M sodium bisulfite solution prior to incubation at 80°C for 40 minutes for bisulfite treatment. DNA was purified from the solutions after bisulfite treatment using the Qiaquick PCR purification kit (QIAGEN) to prepare analysis samples.

### 2. Sequence analysis

PCR was carried out with the following primer sets and DNA in the obtained analysis samples as a template.

### (i) Preparation of PCR reaction solution

A reaction solution (15 µl) was prepared by mixing the following reagents.

| | |
|---|---|
| 10 x Ex Taq^{®} buffer (TAKARA Bio) | 1.5 µl |
| dNTP mix (2.5 mM) | 1.2 µl |
| F primer (10 µM) | 0.6 µl |
| R primer (10 µM) | 0.6 µl |
| Analysis sample (template) | 1.0 µl |
| Ex Taq^{®} polymerase (TAKARA Bio) | 0.12 µl |
| Distilled water | 9.98 µl |
| Total | 15.0 µl |

### (ii) Primer sequences and reaction conditions

(Primer sequences for amplifying the base sequence SEQ ID NO: 15)
F: 5' - TAGTTAGGGATAAAGTGTGAGTGTTA -3' (SEQ ID NO: 54)
R: 5' - CAACTTATTAAATAACTATACTTCCCC -3' (SEQ ID NO: 55)

### (Reaction condition)

1 cycle of 95°C for 4.5 minutes;
40 cycles of 95°C for 30 seconds, 55°C for 15 seconds and 72°C for 30 seconds;
1 cycle of 72°C for 7 minutes; and
keep at 4°C.

(Primer sequences for amplifying the base sequence SEQ ID NO: 16)
F: 5'- GGGATATTTGTTTTTTATATTTAATAAAGT -3' (SEQ ID NO: 56)
R: 5'- ACCTCACAATAAAACTACTACAACC -3' (SEQ ID NO: 57)

### (Reaction condition)

1 cycle of 95°C for 4.5 minutes;
40 cycles of 95°C for 30 seconds, 56°C for 15 seconds and 72°C for 40 seconds;
1 cycle of 72°C for 7 minutes; and
keep at 4°C.

(Primer sequences for amplifying the base sequence SEQ ID NO: 17)
F: 5' - AGGTATTTATGGGGTAGGAATTATA -3' (SEQ ID NO: 58)
R: 5' - AAATCACTTAACTAAAATCCCACTA -3' (SEQ ID NO: 59)

### (Reaction condition)

1 cycle at 95°C for 4.5 minutes;
40 cycles of 95°C for 30 seconds, 56°C for 15 seconds and 72°C for 40 seconds;
1 cycle of 72°C for 7 minutes; and
keep at 4°C.

The obtained PCR products were incorporated into the pCR^{®} 2.1 vector included in the TA cloning kit (Invitrogen). Competent cells (TOP10; Invitrogen) were transformed with the obtained plasmid constructs, and the plasmids were extracted and purified from the transformants by using the GenElute Plasmid Miniprep kit (SIGMA). The base sequences of the PCR products included in the obtained plasmids were analyzed with the Applied Biosystems 3730x1 DNA Analyzer (Applied Biosystems).

After the sequencing, methylation status of CpG sites in the base sequences of the above SEQ ID NOs was analyzed.

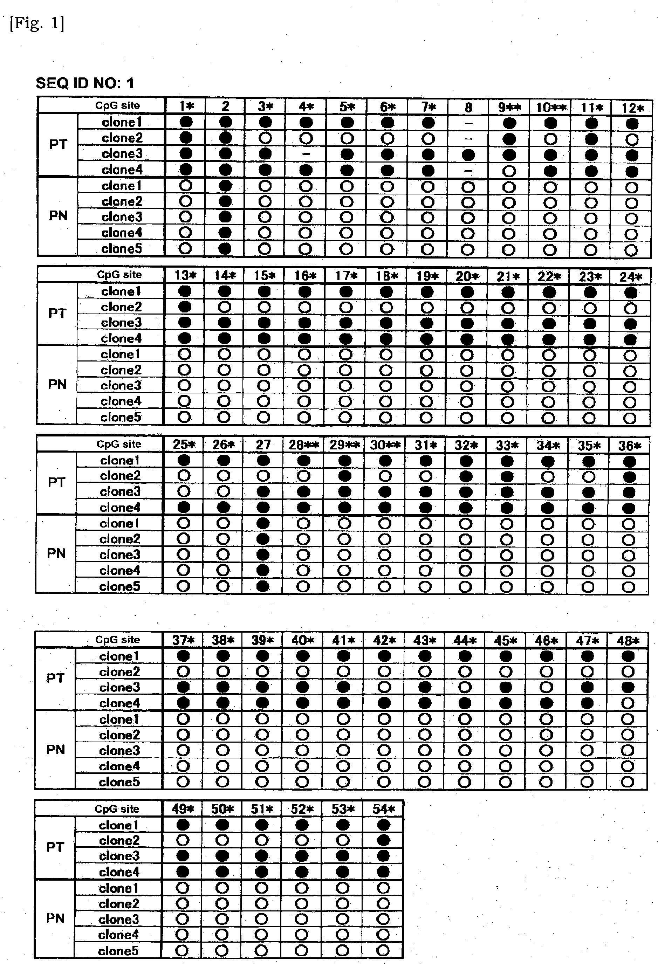

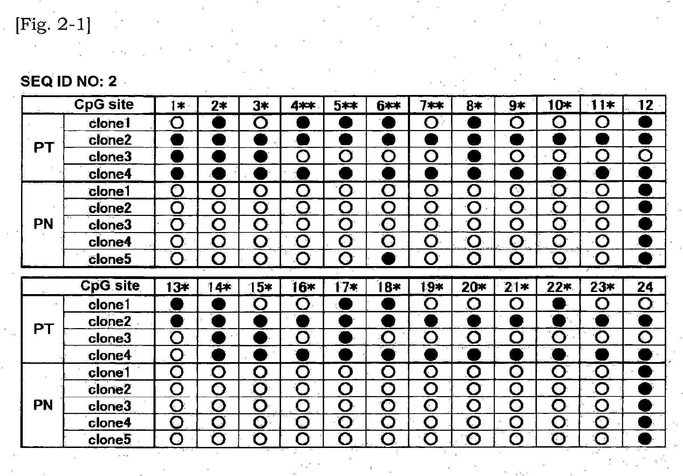

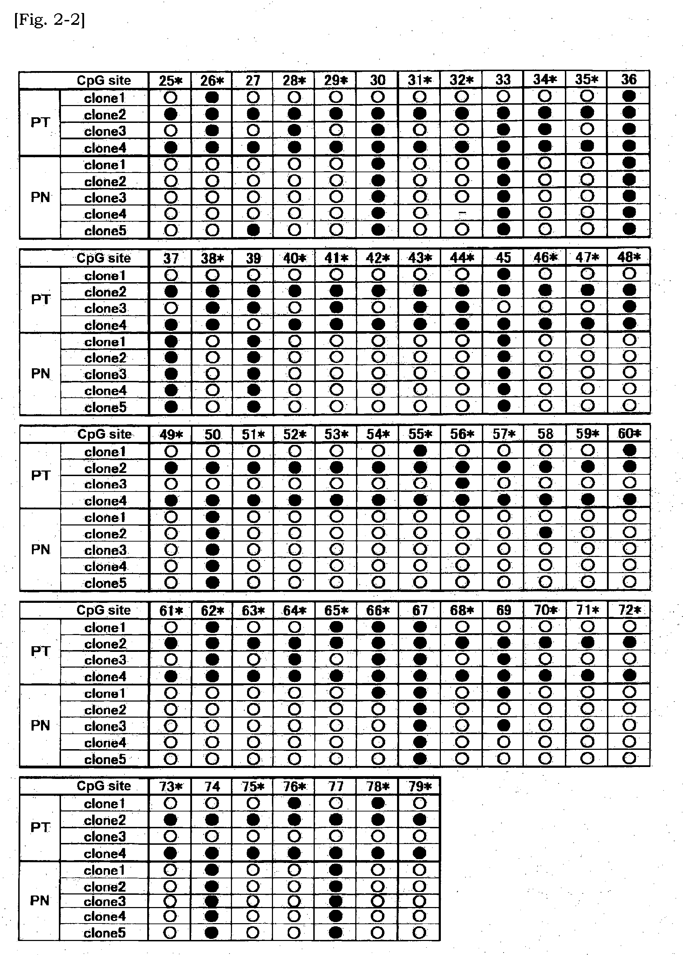

Methylation status of CpG sites located in the base sequences SEQ ID NOs: 1 to 3 which are respectively included in the base sequences SEQ ID NOs: 15 to 17 is shown in the tables in Figs. 1 to 3, which is based on the sequencing results described above. In the tables in these Figures, "●", "○" and "-" represent a methylated CpG site, a non-methylated CpG site and an unanalyzable CpG site, respectively. In addition, "PT" and "PN" represent pancreatic cancer tissue and normal pancreatic tissue, respectively.

In the tables in these Figures, the numbers in the rows correspond to the numbers representing the position of the CpG sites from the 5' end of the respective base sequences SEQ ID NOs: 1 to 3. The CpG site with the symbol "*" or "**" on the number is the CpG site which tends to be methylated in the pancreatic cancer tissue samples compared to the normal pancreatic tissue samples.

Figs. 1 to 3 show that in all base sequences SEQ ID NOs: 1 to 3, CpG sites tend to be methylated in pancreatic cancer tissue but not in normal pancreatic tissue.

Based on these results, methylation frequency in each of the base sequences SEQ ID NOs: 1 to 3 was analyzed. For example, in the case of the base sequence SEQ ID NO: 1, methylation frequency in pancreatic cancer tissue (PT) is the value calculated by dividing the number of methylated CpG sites "●" in all clones (clones 1 to 4) by the sum of the number of methylated CpG site "●" in all clones and the number of non-methylated CpG site "○" and converting the solution into percentage. Similarly, methylation frequency in normal pancreatic tissue (PN) is the value calculated by dividing the number of methylated CpG sites "●" in all clones (clones 1 to 5) by the sum of the number of methylated CpG site "●" in all clones and the number of non-methylated CpG site "○" and converting the solution into percentage.

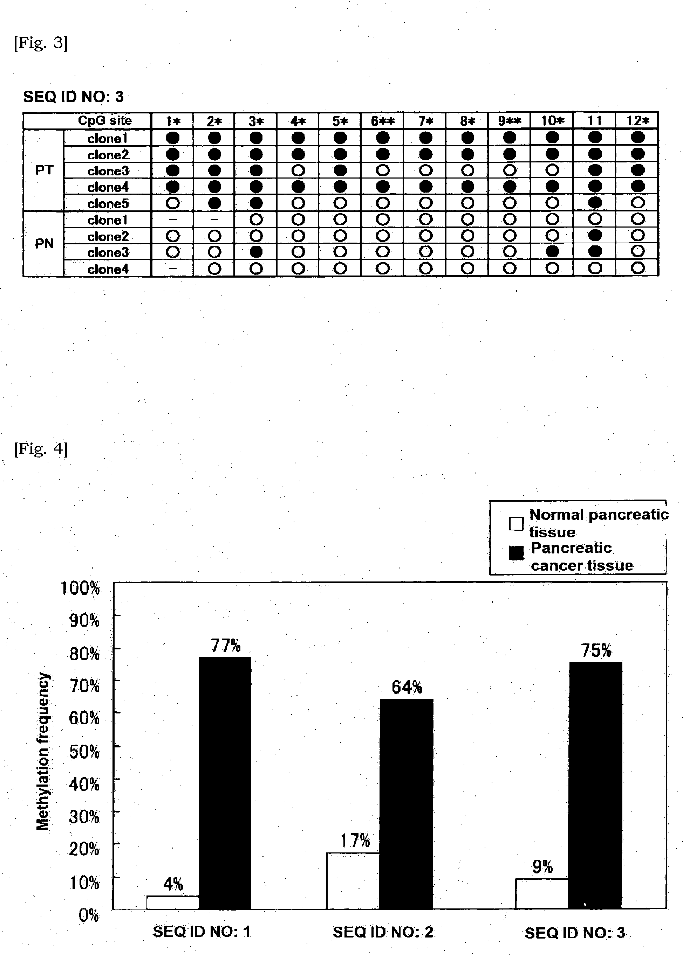

Methylation frequency in the respective base sequences obtained as above is represented as a bar graph in Fig. 4. This graph shows the average values for the group of the pancreatic cancer tissue samples and the group of the normal pancreatic tissues.

Fig. 4 shows that, when all CpG sites located in the base sequences SEQ ID NOs: 1 to 3 are used as targets for analysis, a threshold for differentiating between the samples containing a cancer cell and those devoid of cancer cells can be selected from the range of 20 to 60%, for example.

Accordingly, it was suggested that, when methylation frequency of a CpG site in the base sequence(s) SEQ ID NO(s): 1, 2 and/or 3 in DNA extracted from a biological sample obtained from a subject is higher than the above threshold, it can be determined that a cancer cell is present in the biological sample.

As the present molecular marker for determination, a CpG site which tends to be methylated in cancer tissue but not in normal tissue is suitable.

Accordingly, when presence or absence of a cancer cell in a sample is determined based on methylation frequency of a CpG site in the base sequences SEQ ID NOs: 1 to 3, more accurate determination can be made when the CpG sites(s) as the molecular marker(s) for determination is selected from preferably the CpG site(s) with "*" or "**", more preferably the CpG site(s) with "**" in Figs. 1 to 3.

When presence or absence of a cancer cell in a sample is determined based on the analysis result on presence or absence of methylation of a CpG site(s) in the base sequences SEQ ID NOs: 1 to 3 by methylation specific PCR, more accurate determination can be made when primers are used which can amplify preferably the CpG site(s) with "*" or "**" more preferably the CpG site(s) with "**" in Figs. 1 to 3.

### Example 3: Analysis of methylation status by methylation specific PCR (MSP)

Methylation status of CpG sites located in the gene regions of SEQ ID NOs: 15 to 19 identified in Example 1 was studied.

### 1. Bisulfite treatment

Genomic DNA (2 µg each) obtained from human pancreatic cancer tissue, human normal pancreatic tissue, human normal heart tissue, human normal kidney tissue, human normal liver tissue, human normal lung tissue and human normal peripheral blood leukocytes was subjected to bisulfite treatment in the similar manner as Example 2. DNA was purified from the solutions obtained after bisulfite treatment with the Qiaquick PCR purification kit (QIAGEN) to obtain analysis samples.

### 2. MSP

MSP was carried out with the following primer sets and DNA in the obtained analysis samples as a template. In this MSP, PCR products can be obtained when the template DNA contains a methylated CpG site. In this Example, the CpG sites to be analyzed by MSP are the CpG sites with "**" in Figs. 1 to 3.

### (i) Preparation of PCR reaction solution

A reaction solution (25 µl) was prepared by mixing the following reagents.

2 x FastStart Universal SYBR Green Master (ROX) (ROCHE) 12.5 µl

| | |
|---|---|
| F primer (10 µM) | 1.0 µl |
| R primer (10 µM) | 1.0 µl |
| Analysis sample (template) | 1.0 µl |
| Distilled water | 9.5 ul |
| Total | 25.0 µl |

The sample which contains distilled water instead of the template and the sample which did not contain the template were used as negative controls.

### (ii) Primer sequences and reaction conditions

(Primer sequences for amplifying the base sequence SEQ ID NO: 15)
F: 5'- CGTGGGTTTTAGTTAGGAGC -3' (SEQ ID NO: 28)
R: 5'- ATCCAAAACGACGATATTTAACG -3' (SEQ ID NO: 29)

This primer set analyses the 9th, 10th and 28th to 30th CpG sites from the 5' end of the base sequence SEQ ID NO: 15 (the same positions in the base sequence SEQ ID NO: 1).

### (Reaction condition)

1 cycle of 95°C for 9.5 minutes;
35 cycles of 95°C for 30 seconds, 54°C for 15 seconds and 72°C for 30 seconds; and
keep at 4°C.

(Primer sequences for amplifying the base sequence SEQ ID NO: 16)
F: 5'- CGTTTCGTTCGTATTTATAATAGACG -3' (SEQ ID NO: 30)
R: 5'- AAAACCCATTCTTCCTAACTCCG -3' (SEQ ID NO: 31)

This primer set analyses the 7th to 10th and 20th CpG sites from the 5' end of the base sequence SEQ ID NO: 16 (the 4th to 7th and 17th CpG sites from the 5' end of the base sequence SEQ ID NO: 2).

### (Reaction condition)

1 cycle of 95°C for 9.5 minutes;
40 cycles of 95°C for 30 seconds, 61°C for 15 seconds and 72°C for 30 seconds; and
keep at 4°C.

(Primer sequences for amplifying the base sequence SEQ ID NO: 17)
F: 5'- TGAGGAATGTTAGTAGTAAGGTTACGT -3' (SEQ ID NO: 32)
R: 5'- CCTATATACTCAAAAAAACCACGTC -3' (SEQ ID NO: 33)

This primer set analyses the 9th and 12th CpG sites from the 5' end of the base sequence SEQ ID NO: 17 (the 6th and 9th CpG sites from the 5' end of the base sequence SEQ ID NO: 3).

### (Reaction condition)

1 cycle of 95°C for 9.5 minutes;
40 cycles of 95°C for 30 seconds, 62°C for 15 seconds and 72°C for 30 seconds; and
keep at 4°C.

(Primer sequences for amplifying the base sequence SEQ ID NO: 18)
F: 5'- GTTGGGTTATACGTCGTAGGGC -3' (SEQ ID NO: 34)
R: 5'- GACAAACGAAAATAAACGTCGAA -3' (SEQ ID NO: 35)

This primer set analyses the 21 st to 23rd and 36th to 39th CpG sites from the 5' end of the base sequence SEQ ID NO: 18 (the 1st to 3rd from the 5' end of the base sequence SEQ ID NO: 4 and the 1st to 4th CpG sites from the 5' end of the base sequence SEQ ID NO: 5).

### (Reaction condition)

1 cycle of 95°C for 9.5 minutes;
40 cycles of 95°C for 30 seconds, 57°C for 15 seconds and 72°C for 30 seconds; and
keep at 4°C.

(Primer sequences for amplifying the base sequence SEQ ID NO: 19)
F: 5'- GATAGTTTTAGAGTCGGGGAAGC -3' (SEQ ID NO: 36)
R: 5'- CCTAATCCTAACAAAATCTACCGAC -3' (SEQ ID NO: 37)

This primer set analyses the 74th and 75th CpG sites from the 5' end of the base sequence SEQ ID NO: 19 (the 1st and 2nd CpG sites from the 5' end of the base sequence SEQ ID NO: 6).

### (Reaction condition)

1 cycle of 95°C for 9.5 minutes;
40 cycles of 95°C for 30 seconds, 60°C for 15 seconds and 72°C for 30 seconds; and
keep at 4°C.

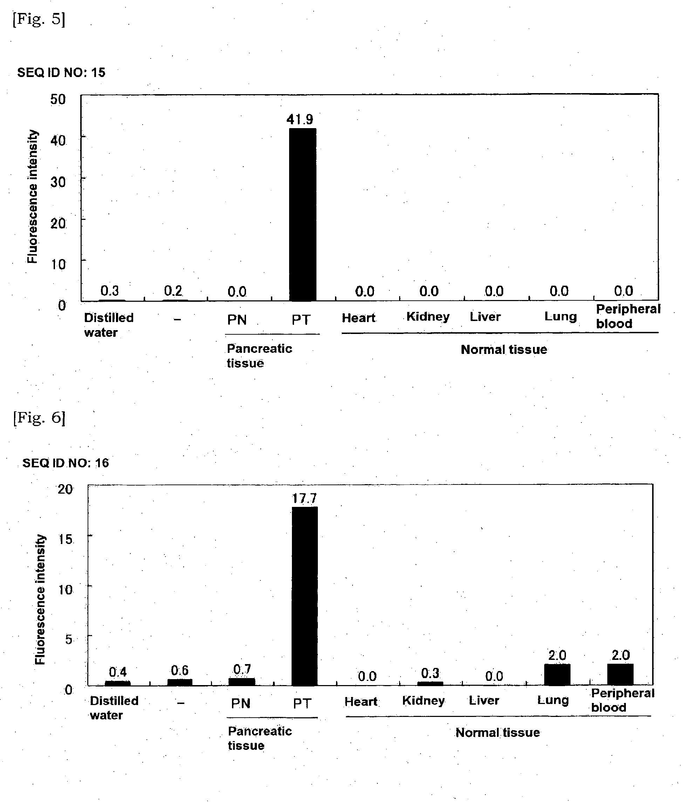

After completion of PCR, the reaction solutions were subjected to electrophoresis on 2% agarose gels, and presence of PCR products was determined based on the bands in the gels. The gels were photographed and the photographs were analyzed with the Quantity One (Bio-Rad) software to quantitatively obtain fluorescence intensity of the bands. The results are shown in Figs. 5 to 9. In these Figures, "-" "PN" and "PT" mean a negative control without the template, normal pancreatic tissue, and pancreatic cancer tissue, respectively.

In MSP for the base sequences SEQ ID NOs: 15 and 16, almost no band was detected in normal tissues (pancreas, heart, kidney, liver, lung and peripheral blood leukocytes), while a band having significantly high intensity was detected for pancreatic cancer tissue (Figs. 5 and 6).

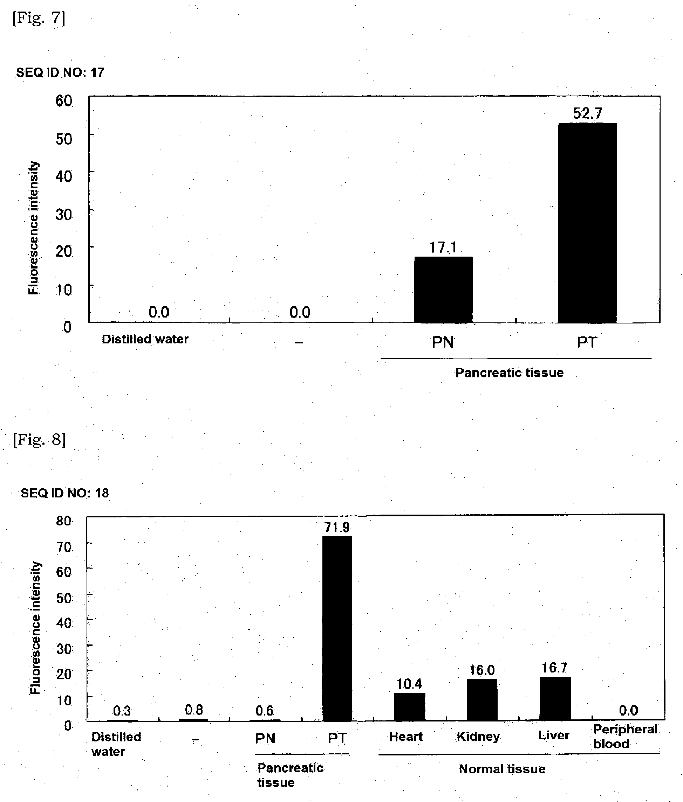

In MSP for the base sequence SEQ ID NO: 17, the intensity of the band detected was significantly in pancreatic cancer tissue than in normal pancreatic tissue (Fig. 7).

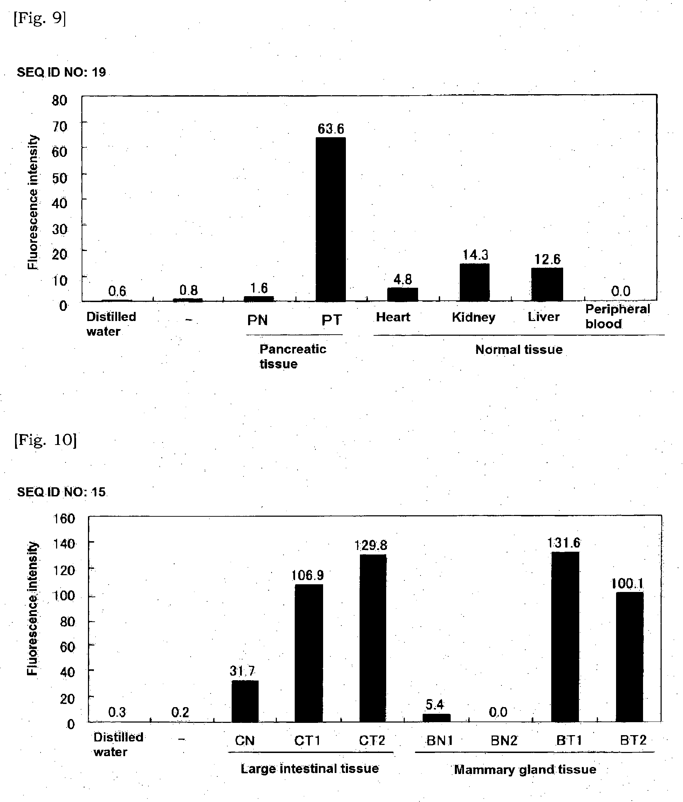

In MSP for the base sequences SEQ ID NOs: 18 and 19, almost no band was detected in normal tissues (pancreas, heart, kidney, liver and peripheral blood leukocytes), while a band having significantly high intensity was detected for pancreatic cancer tissue (Figs. 8 and 9).

As described above, in MSP for the base sequences SEQ ID NOs: 15 to 19, a higher amount of PCR products specific for pancreatic cancer tissue was obtained. This indicates that for genomic DNA from each tissue, MSP analysis on presence or absence of a methylated CpG in the base sequences SEQ ID NOs: 15 to 19, i.e. the base sequences SEQ ID NOs: 1, 2, 3, 4 and 5 and 6 allows differentiation between pancreatic cancer tissue and normal pancreatic tissue.

Accordingly, it is suggested that presence or absence of a cancer cell in a biological sample obtained from a subject can be determined by analyzing presence or absence of methylation of a CpG site located in the present molecular markers, the base sequences SEQ ID NOs: 1, 2, 3, 4, 5 and/or 6 by MSP for DNA extracted from a biological sample obtained from a subject.

### Example 4: Analysis of methylation status in other tissues by MSP

It was studied whether or not a methylated CpG site can be detected for DNA obtained from cancer tissues other than pancreatic cancer tissue.

### 1. Bisulfite treatment

Genomic DNA (2 µg each) obtained from human breast cancer tissue, human normal mammary gland tissue, human colon cancer tissue and human normal large intestinal tissue was subjected to bisulfite treatment in the similar manner as Example 2. DNA was purified from the solutions obtained after bisulfite treatment with the Qiaquick PCR purification kit (QIAGEN) to obtain analysis samples.

### 2. MSP

MSP was carried out with the primer sets having the base sequences SEQ ID NOs: 28 and 29 and SEQ ID NOs: 34 and 35 as above and DNA in the obtained analysis samples as a template. MSP was carried out in the same manner as Example 3.

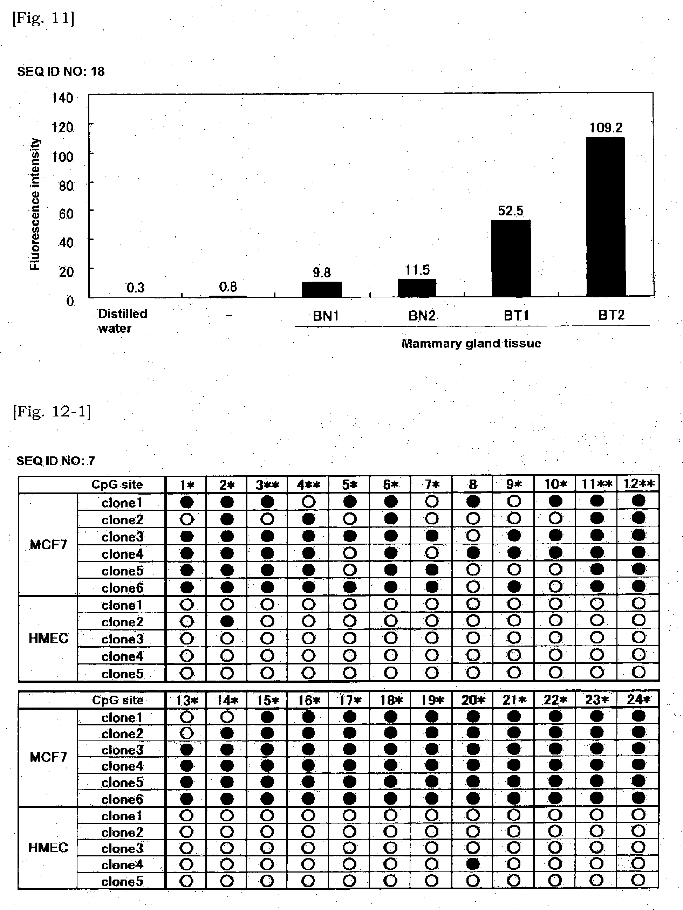

After completion of PCR, the reaction solutions were subjected to electrophoresis on 2% agarose gels in the same manner as Example 3, and fluorescence intensity of the bands appeared in the gels was quantitatively obtained. The results are shown in Figs. 10 and 11. In these Figures, "-" means a negative control without the template, "PN1" and "PN2" mean normal mammary gland tissues, "BT1" and "BT2" mean breast cancer tissues, "CN" means normal large intestinal tissue and "CT1" and "CT2" mean colon cancer tissues.

In MSP for the base sequence SEQ ID NO: 15 using the primer set having the base sequences SEQ ID NOs: 28 and 29, almost no band was detected in normal mammary gland tissue and normal large intestinal tissue, while bands having significantly high intensity were detected for breast cancer tissue and colon cancer tissue (Fig. 10).

In MSP for the base sequence SEQ ID NO: 18 using the primer set having the base sequences SEQ ID NOs: 34 and 35, the intensity of the band detected was significantly higher in breast cancer tissue than in normal mammary gland tissue (Fig. 11).

As described above, in MSP for the base sequences SEQ ID NOs: 15 and 18, a higher amount of PCR products specific for breast cancer tissue and colon cancer tissue was obtained. This indicates that for genomic DNA from each tissue, MSP analysis on presence or absence of a methylated CpG in the base sequences SEQ ID NOs: 15 and 18, i.e. the base sequences SEQ ID NOs: 1 and 4 and 5 allows differentiation between cancer tissue and normal tissue.

Accordingly, it is suggested that presence or absence of a cancer cell can be determined for tissues other than pancreas tissue by analyzing presence or absence of methylation of a CpG site located in the present molecular markers identified from pancreatic cancer tissue by MSP.

### Example 5: Search for molecular markers for determination from breast cancer cell lines

By analyzing genomic DNA obtained from breast cancer cell lines and normal mammary gland epithelial cells with microarrays, gene regions were searched which were methylated specifically in the genomic DNA of the cell lines derived from breast cancer.

The specific procedures in Example 5 followed the instructions attached to the kits and reagents used.

### 1. Search for methylated genes by MeDIP on chip method

### (1) Preparation of methylated DNA by MeDIP method

Genomic DNA was extracted and single-stranded DNA fragments were prepared in the similar manner as Example 1 except that the breast cancer cell lines MCF7, MB-MDA231 and SK-BR-3 and the normal mammary gland epithelial cell line HMEC were used as biological samples.

The obtained single-stranded DNA fragments were diluted in the similar manner as Example 1 followed by addition of Protein G Sepharose beads (68 µl: GE Healthcare). Pre-clear treatment was then carried out in the similar manner as Example 1. The supernatants were collected and then respectively divided into two aliquots in separate tubes, one of which was added with an anti-methylated cytosine antibody BI-MECY-0500 (10 µg: Eurogentec) and the other with a normal mouse IgG antibody (4 µg: Santa Cruz), serving as a test sample and a control sample, respectively. These test and control samples were subjected to immunoprecipitation in the similar manner as Example 1 to elute single-stranded DNA fragments.

The obtained DNA solutions were incubated with Proteinase K (Sigma) before purification with the Qiaquick PCR purification kit (QIAGEN).

Quantitative PCR allowed confirming that methylated DNA in the test samples was specifically concentrated.

### 2. Amplification and labeling of nucleic acid in samples

DNA in the test and control samples respectively obtained from MCF-7, MB-MDA231 and SK-BR-3 and HMEC cells was amplified on WT-Ovation™ Pico RNA Amplification System Version 1.0 (NuGEN). The concentration of the amplified nucleic acid was determined by measuring the absorbance (260 nm and 280 nm) of the samples.

The nucleic acid contained in the above amplified test and controls samples was fragmented and biotinylated on FL-Ovation™ cDNA Biotin Module V2 (NuGEN). The nucleic acid obtained from the samples served as the test and control samples for microarray analysis.

### 3. Microarray analysis

### (1) Contact of sample and microarray

The samples for microarray analysis were brought into contact with the GeneChip^{®} Human Promoter 1.0R Array (Affymetrix) to carry out hybridization with probes in the similar manner as Example 1. One same type of microarray was used for each of the test sample and the control sample.

### (2) Analysis of microarray data

The resulting microarray data was analyzed according to the similar procedures as in Example 1. As a result, the regions represented by the base sequences SEQ ID NOs: 20 to 27 were identified as the gene regions which were specifically methylated in genomic DNA of breast cancer cell lines. The base sequences SEQ ID NOs: 20 to 27 are the gene regions or partial base sequence regions in the vicinity thereof of, respectively, *LBX2, PAX9, ADD3, CCDC61, ZIC4, CGB7, TOX* and *TNNI3* and the regions comprising the base sequences SEQ ID NOs: 7, 8, 9, 10, 11, 12, 13 and 14, respectively.

The correspondence relationship between the base sequences SEQ ID NOs: 20 to 27 and SEQ ID NOs: 7 to 14 and the above genes is summarized in the following Table 3.

**[Table 3]**

| **SEQ ID NO:** | Corresponding SEQ ID NO: | **Gene** |
|---|---|---|
| 20 | 7 | LBX2 |
| 21 | 8 | PAX9 |
| 22 | 9 | ADD3 |
| 23 | 10 | CCDC61 |
| 24 | 11 | ZIC4 |
| 25 | 12 | CGB7 |
| 26 | 13 | TOX |
| 27 | 14 | TNNI3 |

### Example 6: Analysis of methylation status by bisulfite sequencing

By using bisulfite sequencing, it was investigated whether CpG sites in the gene regions represented by SEQ ID NOs: 20 to 27 identified in Example 5 were methylated in genomic DNA of a breast cancer cell line and a normal mammary gland epithelial cell line.

### 1. Bisulfite treatment

Genomic DNA (2 µg) extracted from the breast cancer cell line MCF7 and the normal mammary gland epithelial cell line HMEC used in Example 5 was subjected to bisulfite treatment in the same manner as Example 2. DNA was purified from the solutions after bisulfite treatment using the Qiaquick PCR purification kit (QIAGEN) to prepare analysis samples.

### 2. Sequence analysis

PCR was carried out with the following primer sets and DNA in the obtained analysis samples as a template.

### (i) Preparation of PCR reaction solution

A reaction solution (15 µl) was prepared by mixing the same reagents as Example 2.

### (ii) Primer sequences and reaction conditions

(Primer sequences for amplifying the base sequence SEQ ID NO: 20)
F: 5'- GGAAGAGGTTTAAGTGGATTTTTTT -3' (SEQ ID NO: 60)
R: 5'- TTTTCTTTCCAAACCCAACCTA -3' (SEQ ID NO: 61)

### (Reaction condition)

1 cycle of 95°C for 4.5 minutes;
40 cycles of 95°C for 30 seconds, 62°C for 15 seconds and 72°C for 30 seconds;
1 cycle of 72°C for 7 minutes; and
keep at 4°C.

(Primer sequences for amplifying the base sequence SEQ ID NO: 21)
F: 5'- AGTTAGGATTGTGTAATATTAGTTTT -3' (SEQ ID NO: 62)
R: 5'- CACTATACAACCATCAACTACAAC -3' (SEQ ID NO: 63)

### (Reaction condition)

1 cycle of 95°C for 4.5 minutes;
40 cycles of 95°C for 30 seconds, 55.5°C for 15 seconds and 72°C for 40 seconds;
1 cycle of 72°C for 7 minutes; and
keep at 4°C.

(Primer sequences for amplifying the base sequence SEQ ID NO: 22)
F: 5'- GTATATTTTTAGGGAGGAGGGGGG -3' (SEQ ID NO: 64)
R: 5'- CAACCCCTACTTCACCTCCACATA -3' (SEQ ID NO: 65)

### (Reaction condition)

1 cycle of 95°C for 4.5 minutes;
40 cycles of 95°C for 30 seconds, 65.1°C for 15 seconds and 72°C for 30 seconds;
1 cycle of 72°C for 7 minutes; and
keep at 4°C.

(Primer sequences for amplifying the base sequence SEQ ID NO: 23)
F: 5'- TTATTAGGATGAGTTATTGGTTATTT -3' (SEQ ID NO: 66)
R: 5'- ACCTCCCTAACCCCAACTAC -3' (SEQ ID NO: 67)

### (Reaction condition)

1 cycle of 95°C for 4.5 minutes;
40 cycles of 95°C for 30 seconds, 56.8°C for 15 seconds and 72°C for 40 seconds;
1 cycle of 72°C for 7 minutes; and
keep at 4°C.

(Primer sequences for amplifying the base sequence SEQ ID NO: 24)
F: 5'- GTTTGGGTAGTTTATTGGTT -3' (SEQ ID NO: 68)
R: 5'- CTAAAAATTTCTCAACTCCTAACTC -3' (SEQ ID NO: 69)

### (Reaction condition)

1 cycle of 95°C for 4.5 minutes;
40 cycles of 95°C for 30 seconds, 55.7°C for 15 seconds and 72°C for 30 seconds;
1 cycle of 72°C for 7 minutes; and
keep at 4°C.

(Primer sequences for amplifying the base sequence SEQ ID NO: 25)
F: 5'- GAGGTGATATTAAGGATTTTTGGGT -3' (SEQ ID NO: 70)
R: 5'- TACAACTCAACTCCAATAACCACAC -3' (SEQ ID NO: 71)

### (Reaction condition)

1 cycle of 95°C for 4.5 minutes;
40 cycles of 95°C for 30 seconds, 61.5°C for 15 seconds and 72°C for 40 seconds;
1 cycle of 72°C for 7 minutes; and
keep at 4°C.

(Primer sequences for amplifying the base sequence SEQ ID NO: 26)
F: 5'- AATAAGATTTGTTTAGTTTTATTGTTAAAA -3' (SEQ ID NO: 72)
R: 5'- TCTACCTAATACTCACAACCCCTACA -3' (SEQ ID NO: 73)

### (Reaction condition)

1 cycle at 95°C for 4.5 minutes;
40 cycles of 95°C for 30 seconds, 60°C for 15 seconds and 72°C for 30 seconds;
1 cycle of 72°C for 7 minutes; and
keep at 4°C.

(Primer sequences for amplifying the base sequence SEQ ID NO: 27)
F: 5'- GTAGTAGTAGAGTTTGTAGAGGGGTG -3' (SEQ ID NO: 74)
R: 5'- ATAAAAAATACCTATCCAAAAAAAA -3' (SEQ ID NO: 75)

### (Reaction condition)

1 cycle at 95°C for 4.5 minutes;
40 cycles of 95°C for 30 seconds, 50.9°C for 15 seconds and 72°C for 30 seconds;
1 cycle of 72°C for 7 minutes; and
keep at 4°C.

After the completion of PCR, plasmid constructs containing each of the PCR products were prepared in the similar manner as Example 2 and the constructs were sequenced.

After sequencing, methylation status of CpG sites in the base sequences of the above SEQ ID NOs was analyzed.

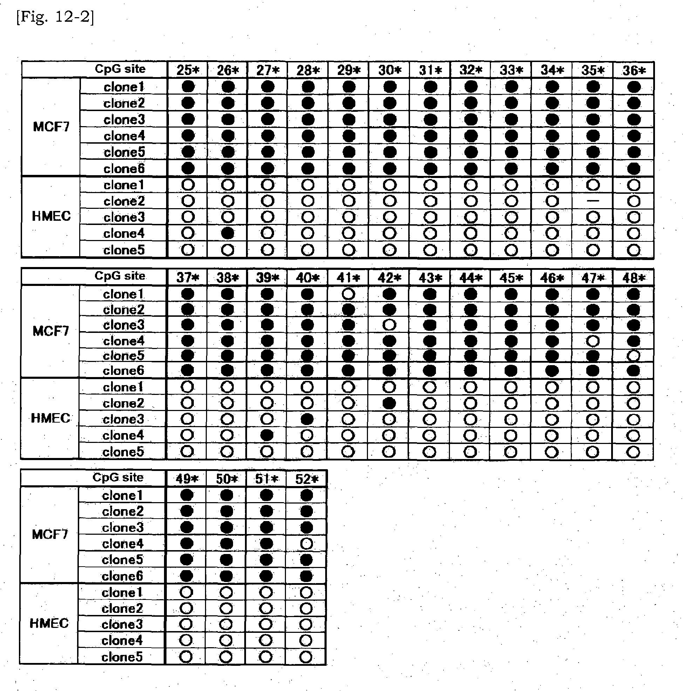

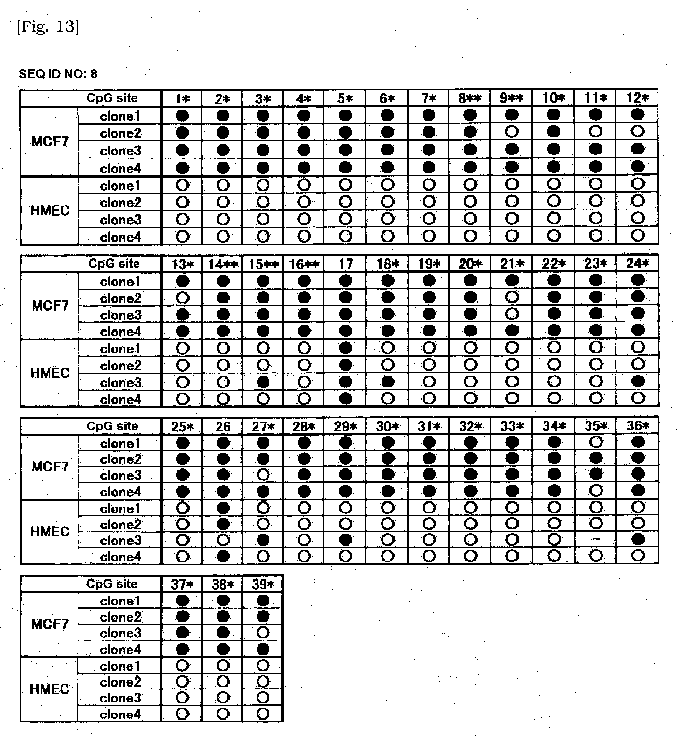

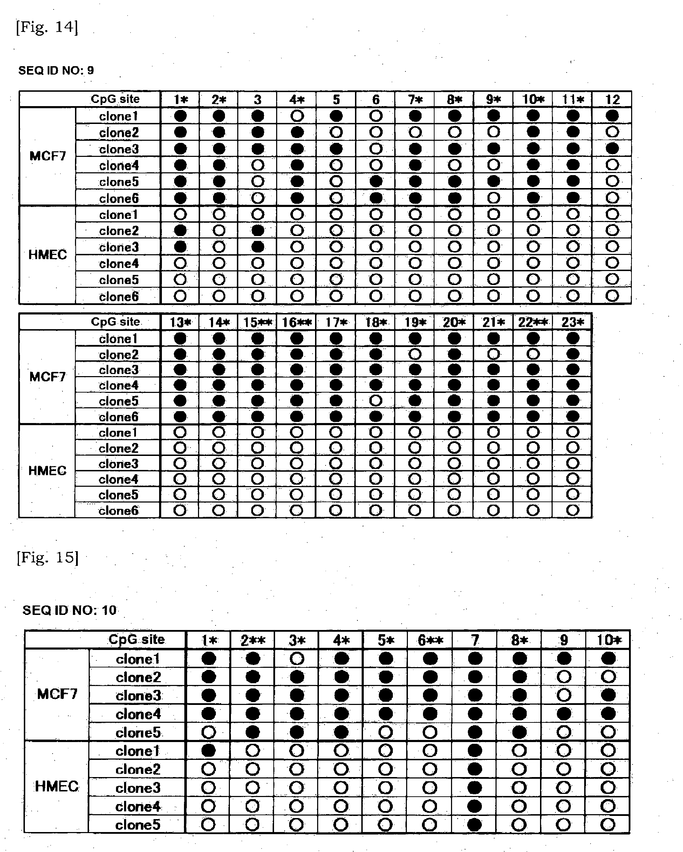

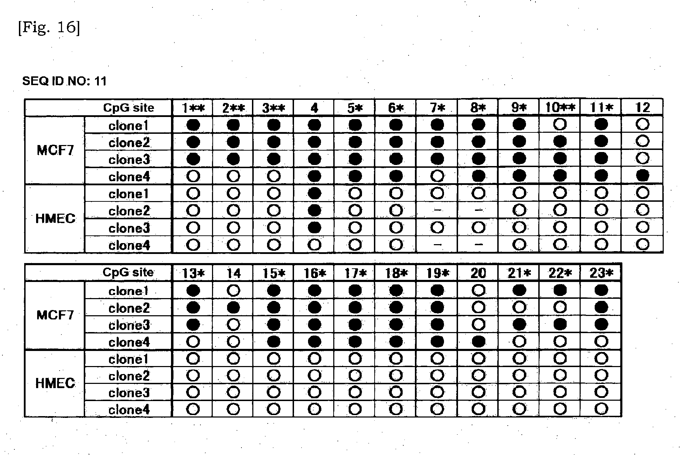

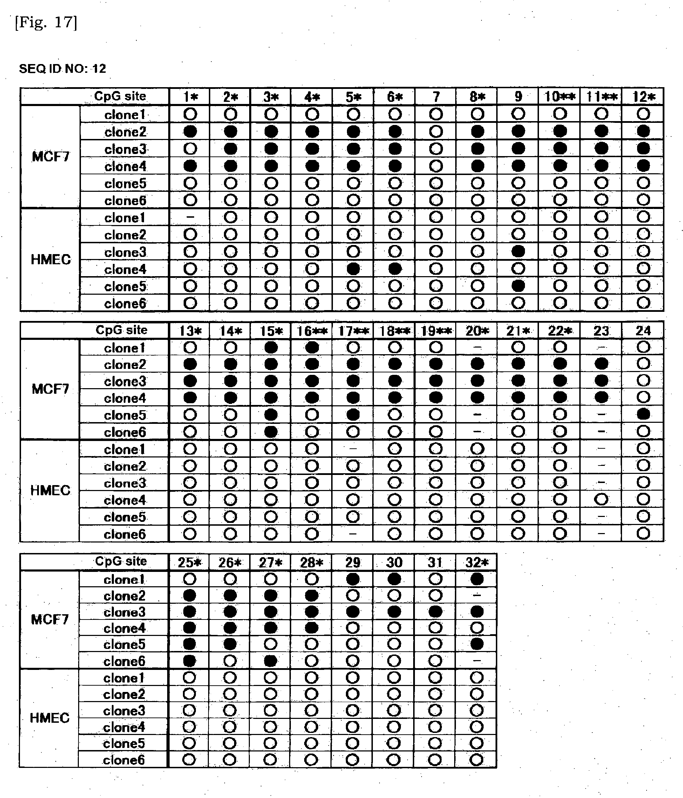

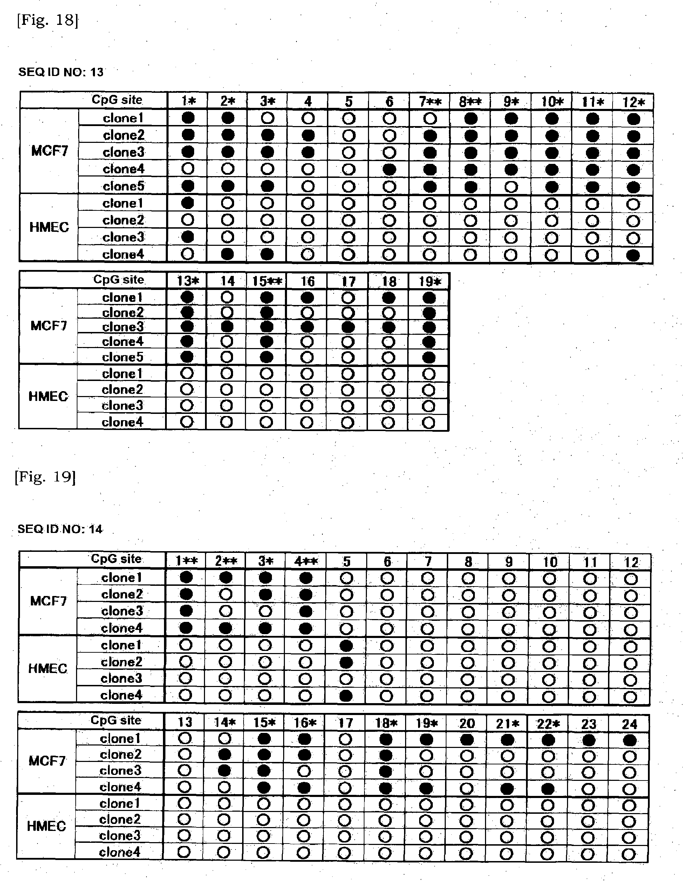

Methylation status of CpG sites located in the base sequences SEQ ID NOs: 7 to 14 which are respectively included in the base sequences SEQ ID NOs: 20 to 27 is shown in the tables in Figs. 12 to 19 based on the results of sequencing described above. In the tables in these Figures, "●", "○" and "-" represent a methylated CpG site, a non-methylated CpG site and an unanalyzable CpG site, respectively.

In the tables in these Figures, the numbers in the rows correspond to the numbers representing the position of the CpG sites from the 5' end of the respective base sequences SEQ ID NOs: 7 to 14. The CpG site with the symbol "*" or "**" on the number is the CpG site which tends to be methylated in the MCF7 cell samples compared to the HMEC cell samples.

Figs. 12 to 19 show that in all base sequences SEQ ID NOs: 7 to 14, CpG sites tend to be methylated in MCF7 cells but not in HMEC cells. Based on these results, methylation frequency in each of the base sequences SEQ ID NOs: 7 to 14 was analyzed. Methylation frequency was calculated in the similar manner as Example 2.

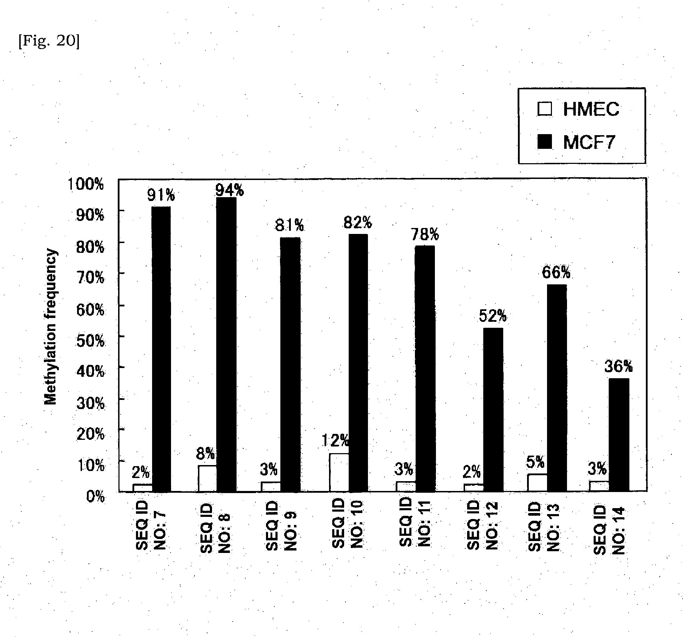

The obtained methylation frequency is shown in the bar graph of Fig. 20. This graph shows the average values for the group of MCF7 cell samples and the group of HMEC cell samples.

Fig. 20 shows that, when all CpG sites located in the base sequences SEQ ID NOs: 7 to 14 are used as targets for analysis, a threshold for differentiating between the samples containing a cancer cell and those devoid of cancer cells can be selected from the range of 15 to 35%, for example.

Accordingly, it was suggested that, when methylation frequency of a CpG site in the base sequence(s) SEQ ID NO(s): 7, 8, 9, 10, 11, 12, 13 and/or 14 in DNA extracted from a biological sample obtained from a subject is higher than the above threshold, it can be determined that a cancer cell is present in the biological sample.

As the present molecular marker for determination, a CpG site which tends to be methylated in cancer cell lines but not in normal cell lines is suitable.

Accordingly, when presence or absence of a cancer cell in a sample is determined based on methylation frequency of a CpG site in the base sequences SEQ ID NOs: 7 to 14, more accurate determination can be made when the CpG site(s) as the molecular marker(s) for determination is selected from preferably the CpG site(s) with "*" or "**", more preferably the CpG site(s) with "**" in Figs. 12 to 19.

When presence or absence of a cancer cell in a sample is determined based on the analysis result on presence or absence of methylation of a CpG site(s) in the base sequences SEQ ID NOs: 7 to 14 by methylation specific PCR, more accurate determination can be made when primers are used which can amplify preferably the CpG site(s) with "*" or "**", more preferably the CpG site(s) with "**" in Figs. 12 to 19.

### Example 7: Analysis of methylation status by MSP

Methylation status of CpG sites located in the gene regions of SEQ ID NOs: 20 to 27 identified in Example 5 was analyzed by MSP for genomic DNA of a breast cancer cell line and a normal mammary gland epithelial cell line.

### 1. Bisulfite treatment

Genomic DNA (2 µg each) obtained from the breast cancer cell line MCF7 and the normal mammary gland epithelial cell line HMEC was subjected to bisulfite treatment in the similar manner as Example 2. DNA was purified from the solutions obtained after bisulfite treatment with the Qiaquick PCR purification kit (QIAGEN) to obtain analysis samples.

### 2. MSP

MSP was carried out with the following primer sets and DNA in the obtained analysis samples as a template. In this Example, the CpG sites analyzed by MSP are the CpG sites with "**" in Figs. 12 to 19. The preparation of the PCR reaction solution was carried out in the similar manner as Example 3.

Primer sequences and reaction conditions are as described hereinbelow.

(Primer sequences for amplifying the base sequence SEQ ID NO: 20)
F: 5'- TTTTAGAGTTTAGGATTGGCGGC -3' (SEQ ID NO: 38)
R: 5'- TACAACTTAACACTACCCGAAAACG -3' (SEQ ID NO: 39)

This primer set analyses the 3rd, 4th, 11th and 12th CpG sites from the 5' end of the base sequence SEQ ID NO: 20 (the same positions in the base sequence SEQ ID NO: 7).

### (Reaction condition)

1 cycle of 95°C for 9.5 minutes;
40 cycles of 95°C for 30 seconds, 62°C for 15 seconds and 72°C for 30 seconds; and
keep at 4°C.

(Primer sequences for amplifying the base sequence SEQ ID NO: 21)
F: 5'- ATAGGTTGGAAACGTAGTTTTTCG -3' (SEQ ID NO: 40)
R: 5'- CTATAACGTCTAACGAATCCTCGC -3' (SEQ ID NO: 41)

This primer set analyses the 8th, 9th and 14th to 16th CpG sites from the 5' end of the base sequence SEQ ID NO: 21 (the same positions in the base sequence SEQ ID NO: 8).

### (Reaction condition)

1 cycle of 95°C for 9.5 minutes;
40 cycles of 95°C for 30 seconds, 62°C for 30 seconds and 72°C for 30 seconds; and
keep at 4°C.

(Primer sequences for amplifying the base sequence SEQ ID NO: 22)
F: 5'- TGTTGTAAAGTTTGTTCGGTTTCGT -3' (SEQ ID NO: 42)
R: 5'- TTCTACTTCATTTAAACCCCTCGAA -3' (SEQ ID NO: 43)

This primer set analyses the 15th, 16th and 22nd CpG sites from the 5' end of the base sequence SEQ ID NO: 22 (the same positions in the base sequence SEQ ID NO: 9).

### (Reaction condition)

1 cycle of 95°C for 9.5 minutes;
40 cycles of 95°C for 30 seconds, 60°C for 15 seconds and 72°C for 30 seconds; and
keep at 4°C.

(Primer sequences for amplifying the base sequence SEQ ID NO: 23)
F: 5'- TGTGTGGAGTAGAATTTTGAGTAAATATGC -3' (SEQ ID NO: 44)
R: 5'- AATTTAAAAACAAAAAAACAACCGCA -3' (SEQ ID NO: 45)

This primer set analyses the 7th and 11 th CpG sites from the 5' end of the base sequence SEQ ID NO: 23 (the 2nd and 6th CpG sites from the 5' end of the base sequence SEQ ID NO: 10).

### (Reaction condition)

1 cycle of 95°C for 9.5 minutes;
35 cycles of 95°C for 30 seconds, 61°C for 15 seconds and 72°C for 30 seconds; and
keep at 4°C.

(Primer sequences for amplifying the base sequence SEQ ID NO: 24)
F: 5'- GTAGTTTATTGGTTCGCGGTC -3' (SEQ ID NO: 46)
R: 5'- AAAAAAAATATATAAAAAAATAACGAT -3' (SEQ ID NO: 47)

This primer set analyses the 1 st to 3rd and 10th CpG sites from the 5' end of the base sequence SEQ ID NO: 24 (the same positions in the base sequence SEQ ID NO: 11).

### (Reaction condition)

1 cycle of 95°C for 9.5 minutes;
40 cycles of 95°C for 30 seconds, 51°C for 15 seconds and 72°C for 30 seconds; and
keep at 4°C.

(Primer sequences for amplifying the base sequence SEQ ID NO: 25)
F: 5'- AGAGTTCGGTTTATTTGGGATAGAATC -3' (SEQ ID NO: 48)
R: 5'- GACCGAAACGTCCTAAACCG -3' (SEQ ID NO: 49)

This primer set analyses the 10th, 11th and 16th to 19th CpG sites from the 5' end of the base sequence SEQ ID NO: 25 (the same positions in the base sequence SEQ ID NO: 12).

### (Reaction condition)

1 cycle of 95°C for 9.5 minutes;
35 cycles of 95°C for 30 seconds, 62°C for 30 seconds and 72°C for 30 seconds; and
keep at 4°C.

(Primer sequences for amplifying the base sequence SEQ ID NO: 26)
F: 5'- TATTGTTTAAGATTCGGAGTTGCGA -3' (SEQ ID NO: 50)
R: 5'- CTCCCAACATTTACCTAATAACGAA -3' (SEQ ID NO: 51)

This primer set analyses the 9th, 10th and 17th CpG sites from the 5' end of the base sequence SEQ ID NO: 26 (the 7th, 8th and 15th CpG sites from the 5' end of the base sequence SEQ ID NO: 13).

### (Reaction condition)

1 cycle of 95°C for 9.5 minutes;
30 cycles of 95°C for 30 seconds, 61°C for 15 seconds and 72°C for 30 seconds; and
keep at 4°C.

(Primer sequences for amplifying the base sequence SEQ ID NO: 27)
F: 5'- GGGAGGGAAGCGTAGTTTATTC -3' (SEQ ID NO: 52)
R: 5'- CTAAAAAATTTAAAAAAACAAAAACGAT -3' (SEQ ID NO: 53)

This primer set analyses the 1st, 2nd and 4th CpG sites from the 5' end of the base sequence SEQ ID NO: 27 (the same positions in the base sequence SEQ ID NO: 14).

### (Reaction condition)

1 cycle of 95°C for 9.5 minutes;
40 cycles of 95°C for 30 seconds, 54°C for 15 seconds and 72°C for 30 seconds; and
keep at 4°C.

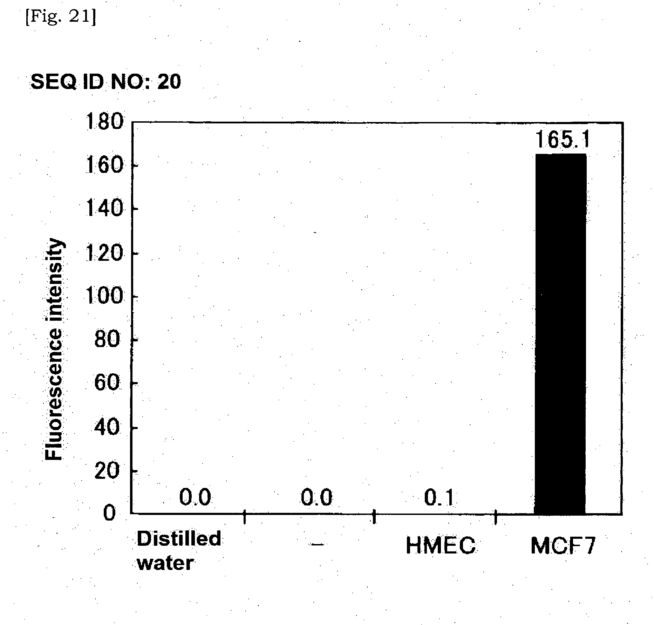

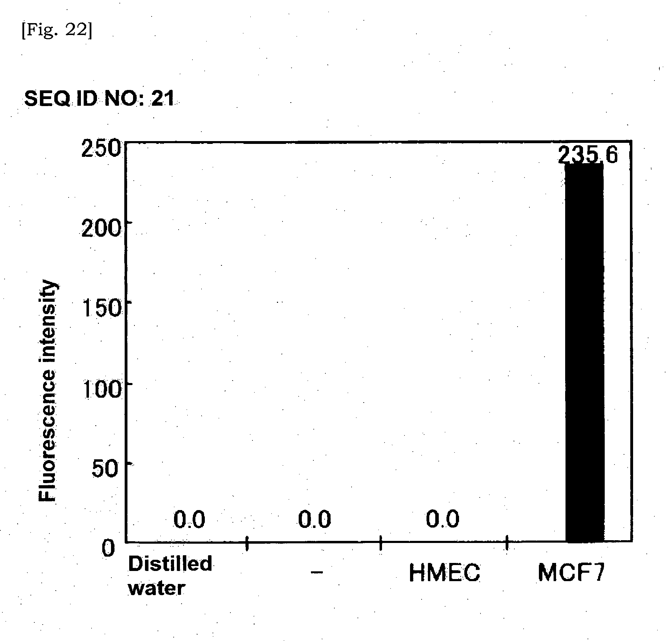

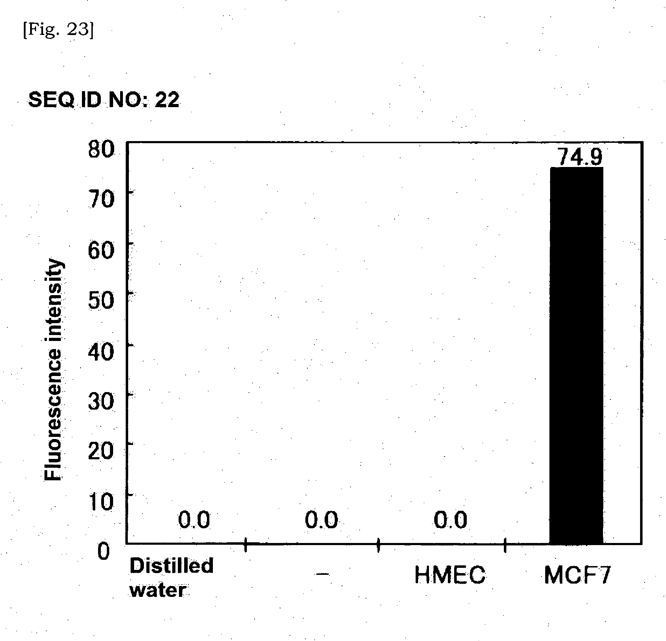

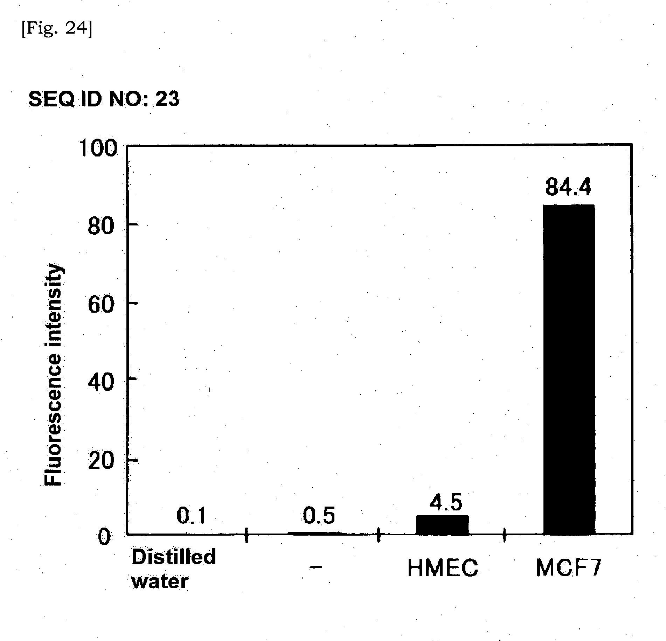

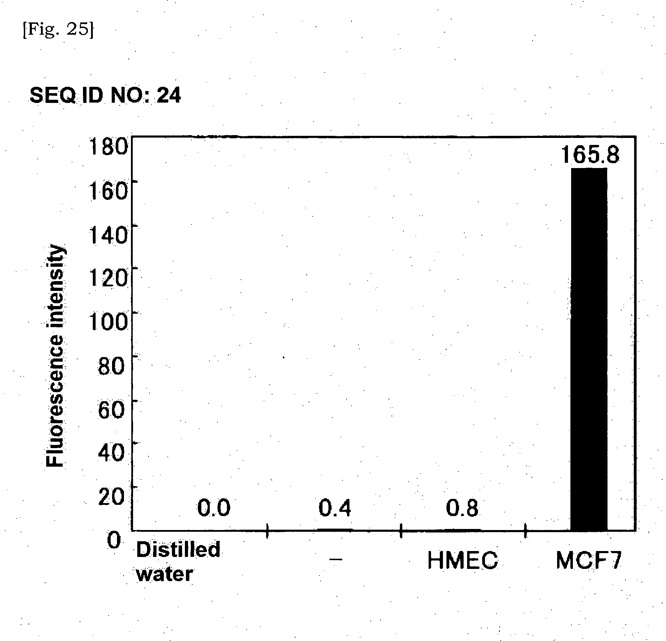

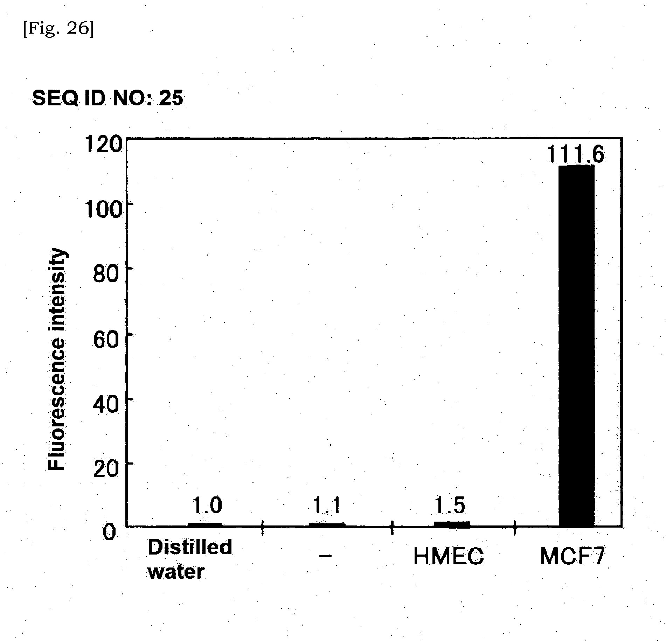

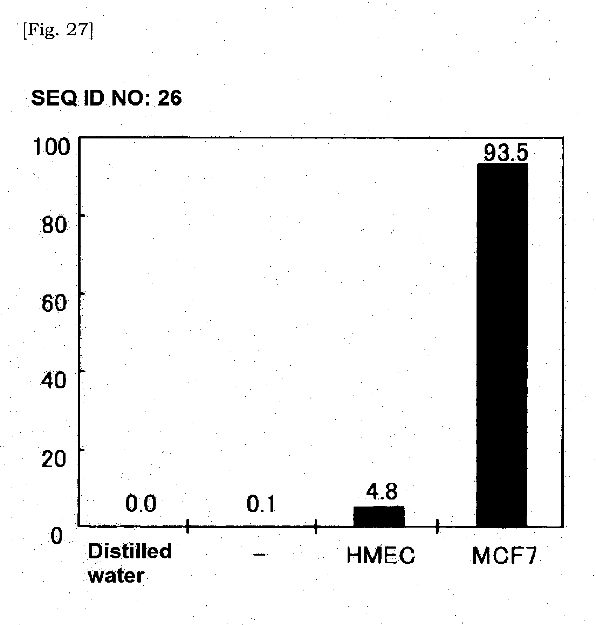

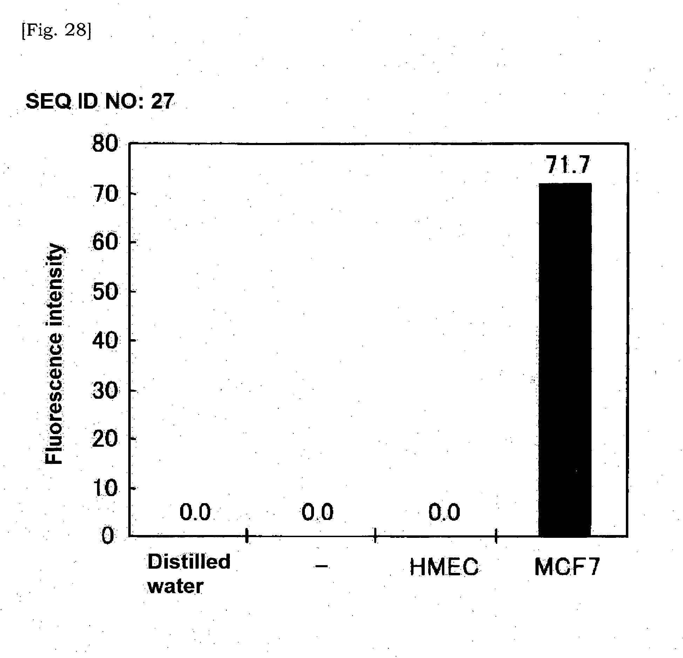

After completion of PCR, the reaction solutions were subjected to electrophoresis on 2% agarose gels in the same manner as Example 3, and fluorescence intensity of the bands appeared in the gels was quantitatively obtained. The results are shown in Figs. 21 to 28. In these Figures, "-" means a negative control without the template.

In MSP for each of the base sequences SEQ ID NOs: 20 to 27, bands having significantly higher intensity were detected for MCF7 cells than for HMEC cells (see Figs. 21 to 28).

As described above, in MSP for the base sequences SEQ ID NOs: 20 to 27, a higher amount of PCR products specific for breast cancer cells was obtained. This indicates that for genomic DNA from each cell line, MSP analysis on presence or absence of a methylated CpG in the base sequences SEQ ID NOs: 20 to 27, i.e. the base sequences SEQ ID NOs: 7 to 14 allows differentiation between breast cancer cells and normal mammary gland epithelial cells.

Accordingly, it is suggested that presence or absence of a cancer cell can be determined by analyzing presence or absence of methylation of a CpG site located in the present molecular marker(s) for determination, i.e. the base sequence(s) SEQ ID NO(s): 7, 8, 9, 10, 11, 12, 13 and/or 14 by MSP for DNA extracted from a biological sample obtained from a subject.

### Example 8: Analysis of methylation status of genomic DNA from breast cancer tissue and normal mammary gland tissue by MSP

By using MSP, it was investigated whether a methylated CpG site could be detected located in the gene regions represented by the base sequences SEQ ID NOs: 20 to 27 for not only a breast cancer cell line but also for breast cancer tissue.

### 1. Bisulfite treatment

Genomic DNA (2 µg each) obtained from human breast cancer tissue and human normal mammary gland tissue was subjected to bisulfite treatment in the same manner as Example 2. DNA was purified from the solutions after bisulfite treatment using the Qiaquick PCR purification kit (QIAGEN) to obtain analysis samples.

### 2. MSP

MSP was carried out with the primer sets having the base sequences SEQ ID NOs: 38 to 53 as above and DNA in the obtained analysis samples as a template. The PCR reaction solution was prepared in the similar manner as Example 3, and the reaction conditions of MSP using the above primer sets are the same as Example 7.

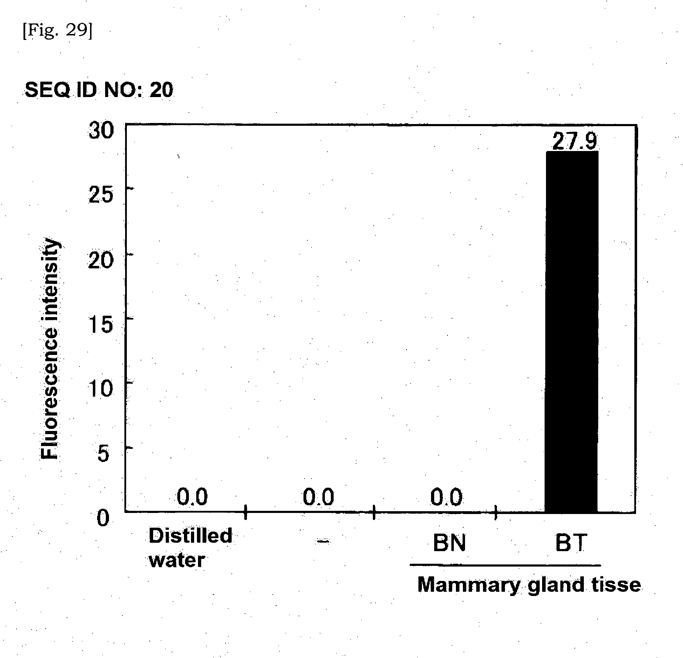

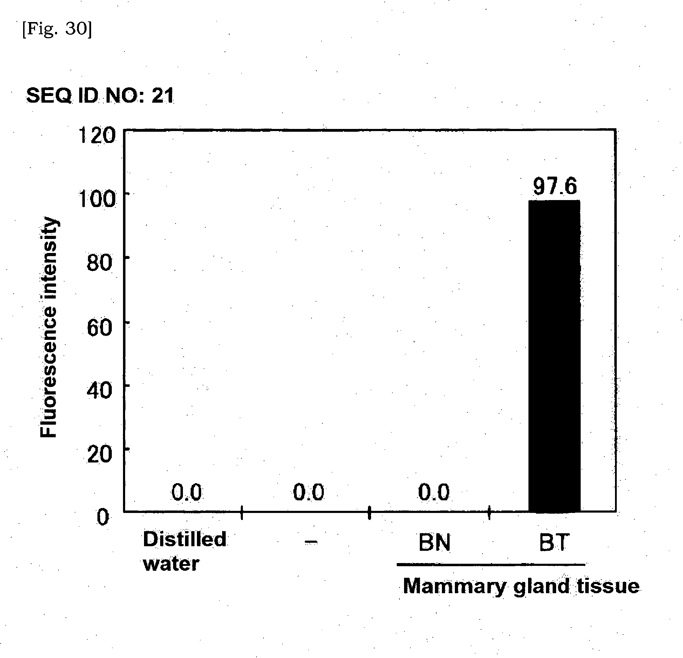

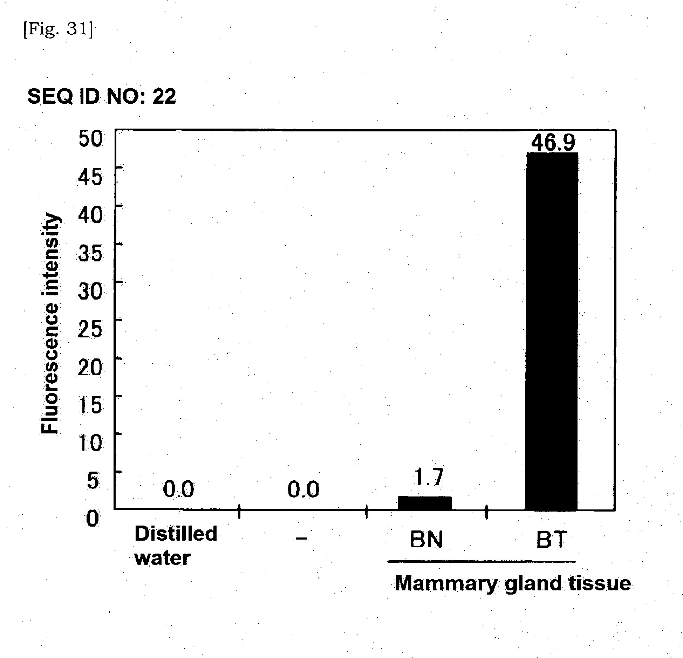

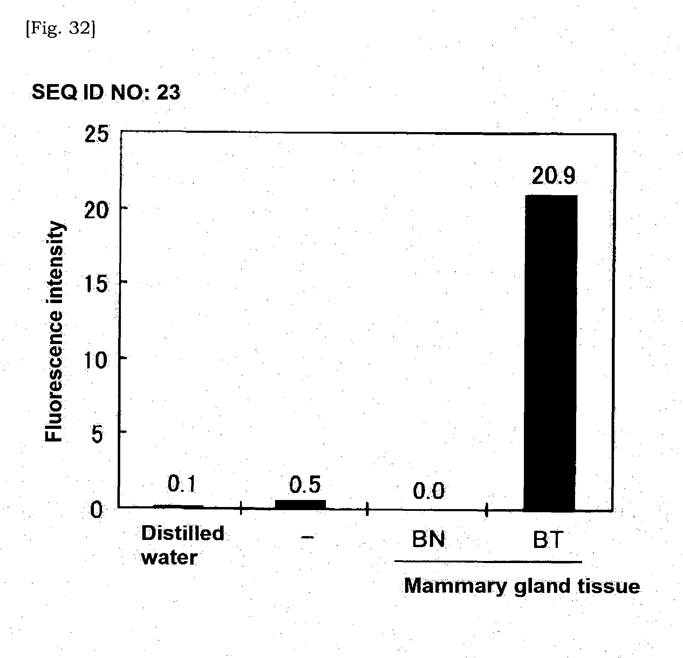

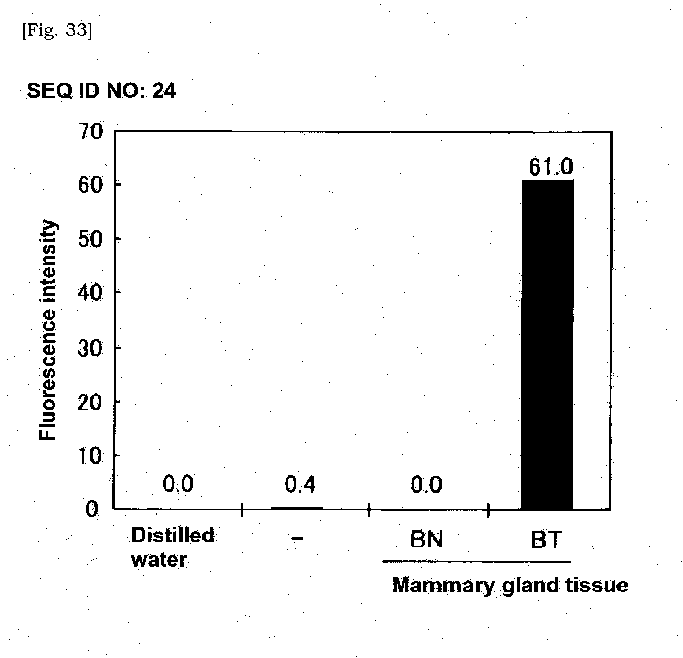

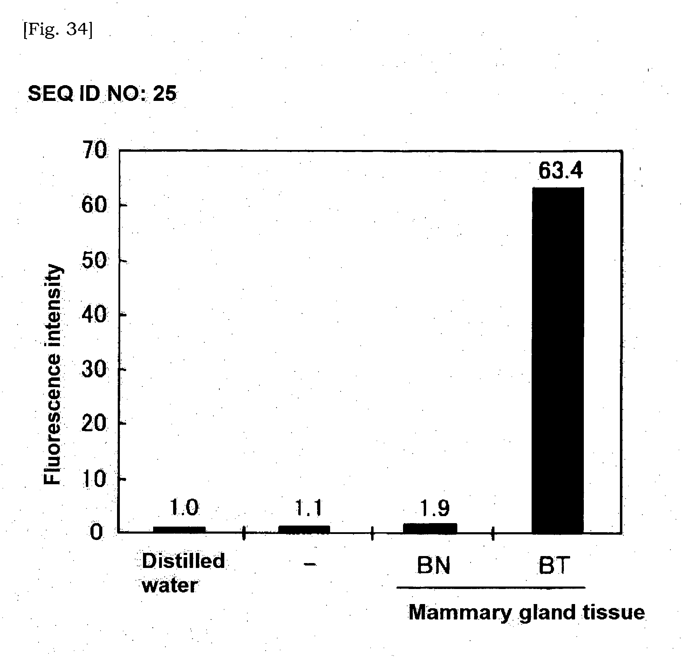

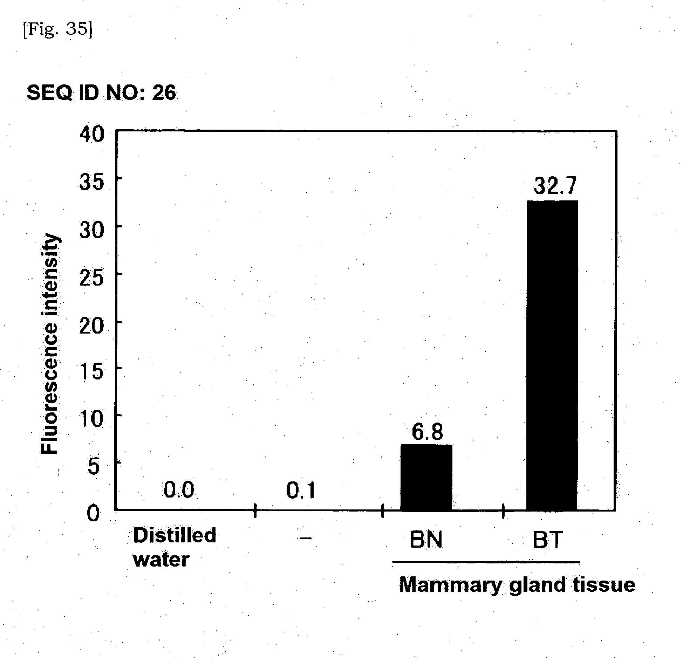

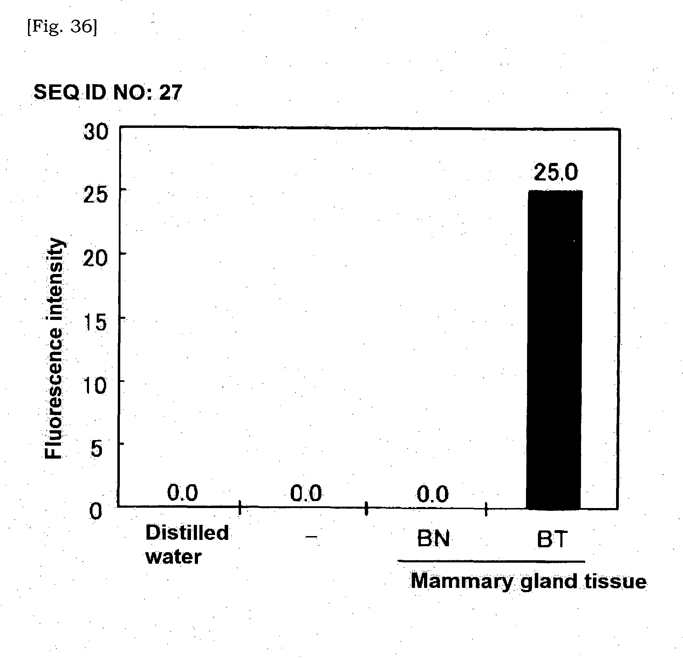

After completion of PCR, the reaction solutions were subjected to electrophoresis on 2% agarose gels in the same manner as Example 3, and fluorescence intensity of the bands appeared in the gels was quantitatively obtained. The results are shown in Figs. 29 to 36. In these Figures, "-" means a negative control without the template.

In MSP for the base sequences SEQ ID NOs: 20 to 27 using the primer sets as above, almost no band was detected in normal mammary gland tissue, while bands having significantly high intensity were detected for breast cancer tissue (see Figs. 29 to 36).

As described above, in MSP for the base sequences SEQ ID NOs: 20 to 27, a higher amount of PCR products specific for breast cancer tissue was obtained. This indicates that for genomic DNA from each tissue, MSP analysis on presence or absence of a methylated CpG in the base sequences SEQ ID NOs: 20 to 27 i.e., the base sequences SEQ ID NOs: 7 to 14 allows differentiation between breast cancer tissue and normal mammary gland tissue.

Accordingly, it is suggested that presence or absence of a cancer cell in tissue obtained from a subject can be determined by analyzing presence or absence of methylation of a CpG site located in the present molecular markers for determination.

### Example 9: Analysis of methylation status of genomic DNA from colon cancer tissue and normal large intestinal tissue by MSP

Methylation status of CpG sites located in the gene regions represented by the base sequences SEQ ID NOs: 17 to 19, 21, 22 and 25 to 27 was studied for genomic DNA of colon cancer tissue and normal large intestinal tissue by MSP.

### 1. Bisulfite treatment

Genomic DNA of human colon cancer tissue and human normal large intestinal tissue (2 µg each: BioChain) was subjected to bisulfite treatment in the similar manner as Example 2. DNA was purified from the solutions obtained after bisulfite treatment with the Qiaquick PCR purification kit (QIAGEN) to obtain analysis samples.

### 2. MSP

MSP was carried out with the primer sets represented by the above SEQ ID NOs: 32 to 37, 40 to 43 and 48th to 53rd and DNA in the obtained analysis samples as a template. PCR reaction solutions were prepared as follows.

### (i) Preparation of PCR reaction solution

A reaction solution (25 µl) was prepared by mixing the following reagents.

2 x FastStart Universal SYBR Green Master (ROX) (ROCHE) 12.5 µl

| | |
|---|---|
| F primer (10 µM) | 1.0 µl |
| R primer (10 µM) | 1.0 µl |
| Analysis sample (template) | 1.0 µl |
| Distilled water | 9.5 ul |
| Total | 25.0 µl |

The reaction conditions of MSP using the above respective primer sets are as follows.

(Reaction condition of MSP using the primer set (SEQ ID NOs: 32 and 33) amplifying the base sequence SEQ ID NO: 17)
1 cycle of 95°C for 9.5 minutes;
40 cycles of 95°C for 30 seconds, 62°C for 15 seconds and 72°C for 30 seconds; and
keep at 4°C.

(Reaction condition of MSP using the primer set (SEQ ID NOs: 34 and 35) amplifying the base sequence SEQ ID NO: 18)
1 cycle of 95°C for 9.5 minutes;
40 cycles of 95°C for 30 seconds, 58°C for 15 seconds and 72°C for 30 seconds; and
keep at 4°C.

(Reaction condition of MSP using the primer set (SEQ ID NOs: 36 and 37) amplifying the base sequence SEQ ID NO: 19)
1 cycle of 95°C for 9.5 minutes;
35 cycles of 95°C for 30 seconds, 60°C for 15 seconds and 72°C for 30 seconds; and
keep at 4°C.

(Reaction condition of MSP using the primer set (SEQ ID NOs: 40 and 41) amplifying the base sequence SEQ ID NO: 21)
1 cycle of 95°C for 9.5 minutes;
40 cycles of 95°C for 30 seconds, 62°C for 15 seconds and 72°C for 30 seconds; and
keep at 4°C.

(Reaction condition of MSP using the primer set (SEQ ID NOs: 42 and 43) amplifying the base sequence SEQ ID NO: 22)
1 cycle of 95°C for 9.5 minutes;
40 cycles of 95°C for 30 seconds, 61°C for 15 seconds and 72°C for 30 seconds; and
keep at 4°C.

(Reaction condition of MSP using the primer set (SEQ ID NOs: 48 and 49) amplifying the base sequence SEQ ID NO: 25)
1 cycle of 95°C for 9.5 minutes;
35 cycles of 95°C for 30 seconds, 61°C for 15 seconds and 72°C for 30 seconds; and
keep at 4°C.

(Reaction condition of MSP using the primer set (SEQ ID NOs: 50 and 51) amplifying the base sequence SEQ ID NO: 26)
1 cycle of 95°C for 9.5 minutes;
35 cycles of 95°C for 30 seconds, 60°C for 15 seconds and 72°C for 30 seconds; and
keep at 4°C.

(Reaction condition of MSP using the primer set (SEQ ID NOs: 52 and 53) amplifying the base sequence SEQ ID NO: 27)
1 cycle of 95°C for 9.5 minutes;
40 cycles of 95°C for 30 seconds, 53°C for 15 seconds and 72°C for 30 seconds; and
keep at 4°C.

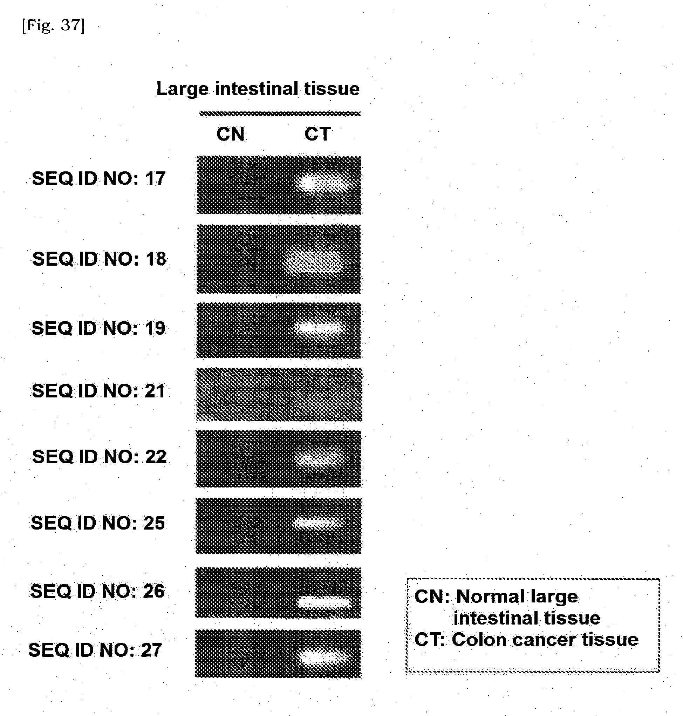

After completion of PCR, the reaction solutions were subjected to electrophoresis on 2% agarose gels, and the presence or absence of the bands for the PCR products was determined. The result is shown in Fig. 37. In this Figure, "CN" and "CT" mean normal large intestinal tissue and colon cancer tissue, respectively.

Fig. 37 shows that in MSP for the base sequences SEQ ID NOs: 17 to 19, 21, 22 and 25 to 27 using the above primer sets, no band was detected for normal large intestinal tissue, and the bands were detected only for colon cancer tissue.

As described above, in MSP for the base sequences SEQ ID NOs: 17 to 19, 21, 22 and 25 to 27, PCR products specific for colon cancer tissue were obtained. This indicates that for genomic DNA from each tissue, MSP analysis on presence or absence of a methylated CpG in the base sequences SEQ ID NOs: 17 to 19, 21, 22 and 25 to 27, i.e. the base sequences SEQ ID NOs: 3 to 6, 8, 9 and 12 to 14 allows differentiation between colon cancer tissue and normal large intestinal tissue.

Accordingly, it is suggested that presence or absence of a cancer cell in tissue other than pancreas tissue and mammary gland tissue can be determined by analyzing presence or absence of methylation of a CpG site located in the present molecular markers identified from pancreatic cancer tissue and breast cancer cell lines by MSP.

### Example 10: Analysis of methylation status of genomic DNA from various types of normal tissue by MSP

Methylation status of CpG sites located in the gene regions represented by the base sequences SEQ ID NOs: 20 to 27 was studied for genomic DNA from various types of normal tissue.

### 1. Bisulfite treatment

Genomic DNA (2 µg each: BioChain) from human normal heart tissue, human normal kidney tissue, human normal liver tissue, human normal lung tissue and human normal peripheral blood leukocytes was subjected to bisulfite treatment in the similar manner as Example 2. DNA was purified from the solutions obtained after bisulfite treatment with the Qiaquick PCR purification kit (QIAGEN) to obtain analysis samples. Genomic DNA from the breast cancer cell line MCF7 was treated in the similar manner as described for the above normal tissues to obtain a positive control sample.

### 2. MSP

MSP was carried out with the primer sets represented by SEQ ID NOs: 38 to 53 as above and DNA in the obtained analysis samples and the positive control sample as a template. PCR reaction solutions were prepared in the similar manner as Example 9.

The reaction conditions of MSP using the above respective primer sets are as follows.

(Reaction condition of MSP using the primer set (SEQ ID NOs: 38 and 39) amplifying the base sequence SEQ ID NO: 20)
1 cycle of 95°C for 9.5 minutes;
40 cycles of 95°C for 30 seconds, 62°C for 15 seconds and 72°C for 30 seconds; and
keep at 4°C.

(Reaction condition of MSP using the primer set (SEQ ID NOs: 40 and 41) amplifying the base sequence SEQ ID NO: 21)
1 cycle of 95°C for 9.5 minutes;
40 cycles of 95°C for 30 seconds, 62°C for 15 seconds and 72°C for 30 seconds; and
keep at 4°C.

(Reaction condition of MSP using the primer set (SEQ ID NOs: 42 and 43) amplifying the base sequence SEQ ID NO: 22)
1 cycle of 95°C for 9.5 minutes;
40 cycles of 95°C for 30 seconds, 61°C for 15 seconds and 72°C for 30 seconds; and
keep at 4°C.

(Reaction condition of MSP using the primer set (SEQ ID NOs: 44 and 45) amplifying the base sequence SEQ ID NO: 23)
1 cycle of 95°C for 9.5 minutes;
35 cycles of 95°C for 30 seconds, 60°C for 15 seconds and 72°C for 30 seconds; and
keep at 4°C.

(Reaction condition of MSP using the primer set (SEQ ID NOs: 46 and 47) amplifying the base sequence SEQ ID NO: 24)
1 cycle of 95°C for 9.5 minutes;
40 cycles of 95°C for 30 seconds, 50°C for 15 seconds and 72°C for 30 seconds; and
keep at 4°C.

(Reaction condition of MSP using the primer set (SEQ ID NOs: 48 and 49) amplifying the base sequence SEQ ID NO: 25)
1 cycle of 95°C for 9.5 minutes;
35 cycles of 95°C for 30 seconds, 61°C for 15 seconds and 72°C for 30 seconds; and
keep at 4°C.

(Reaction condition of MSP using the primer set (SEQ ID NOs: 50 and 51) amplifying the base sequence SEQ ID NO: 26)
1 cycle of 95°C for 9.5 minutes;
35 cycles of 95°C for 30 seconds, 60°C for 15 seconds and 72°C for 30 seconds; and
keep at 4°C.

(Reaction condition of MSP using the primer set (SEQ ID NOs: 52 and 53) amplifying the base sequence SEQ ID NO: 27)
1 cycle of 95°C for 9.5 minutes;
40 cycles of 95°C for 30 seconds, 53°C for 15 seconds and 72°C for 30 seconds; and
keep at 4°C.

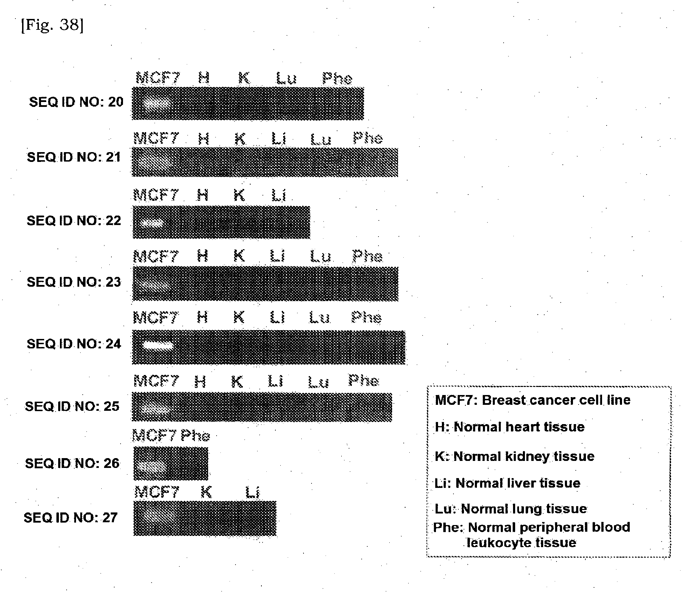

After completion of PCR, the reaction solutions were subjected to electrophoresis on 2% agarose gels, and the presence or absence of the PCR products was determined. The result is shown in Fig. 38. In this Figure, "MCF7" means the breast cancer cell line MCF7, "H" means the normal heart tissue, "K" means the normal kidney tissue, "Li" means the normal liver tissue, "Lu" means the normal lung tissue and "Phe" means the normal peripheral blood leukocytes.

Fig. 38 shows that in MSP for the base sequences SEQ ID NOs: 20 to 27 using the above primer sets, no band was detected for all normal tissues. The above MSP reaction conditions are the ones which sufficiently allow the detection of a band when the positive control sample prepared from genomic DNA of the MCF7 cells was used as a template.

As described above, in MSP for the base sequences SEQ ID NOs: 20 to 27, no PCR product was obtained for various types of normal tissue. This indicates that by MSP analysis of genomic DNA of the normal tissues, methylation of CpG sites located in the base sequences SEQ ID NOs: 20 to 27, i.e. SEQ ID NOs: 7 to 14 is not detected.

Accordingly, it is suggested that analysis of presence or absence of methylation of the CpG site(s) located in the present molecular marker for determination allows determination of absence of a cancer cell in various types of normal tissue.

## Claims

1. A method for determining presence or absence of a cancer cell in a biological sample obtained from a subject comprising the steps of:
extracting DNA from the biological sample;
analyzing methylation status, for the DNA obtained in the extracting step, by performing methylation-specific PCR using a primer set amplifying at least one CpG site chosen from the 9th, 10th and 28th to 30th CpG sites from the 5' end of a base sequence of SEQ ID NO: 1 of the HOXA9 gene; and
determining presence or absence of the cancer cell in the biological sample based on a result of the PCR, wherein it is determined that the cancer cell is present in the biological sample when amplified product of the PCR is detected.

2. The method according to claim 1, wherein the analyzing step comprises concentrating methylated DNA contained in the DNA obtained from the extracting step by immunoprecipitation and analyzing methylation status of the concentrated methylated DNA.

3. The method according to any one of claims 1 to 2, wherein 3' end of one of the primers in said primer set is cytosine.

4. The method according to any one of claims 1 to 4, wherein said primer set comprises the base sequences SEQ ID NO: 28 and SEQ ID NO: 29.

5. The method according to any one of claims 1 to 5, wherein methylation-specific PCR is performed using a primer set amplifying the 9th, 10th and 28th to 30th CpG sites from the 5' end of a base sequence of SEQ ID NO: 1 of the HOXA9 gene.

6. The method according to any one of claims 1 to 5, wherein said biological sample comprises cancer tissue chosen from the group consisting of pancreatic cancer tissue, breast cancer tissue and colon cancer tissue.

7. A primer set consisting of the base sequences SEQ ID NO: 28 and SEQ ID NO: 29.

8. A kit for determination of presence or absence of a cancer cell in a biological sample obtained from a subject comprising:
a non-methylated cytosine conversion agent that converts non-methylated
cytosine in DNA extracted from the bioiogical, sample to a different base; and
a primer set for amplifying by methylation specific PCR the 9th, 10th and 28th to 30th CpG sites from the 5' end of a base sequence of SEQ ID NO: 1 of the HOXA9 gene.

9. In vitro use of a primer set, amplifying at least one CpG site chosen from the 9th, 10th and 28th to 30th CpG sites from the 5' end of a base sequence of SEQ ID NO: 1 of the HOXA9, for determining presence or absence of a cancer cell in a biological sample obtained from a subject, by methylation specific PCR

10. Use of a primer set according to claim 9, amplifying the 9th, 10th and 28th to 30th CpG sites from the 5' end of a base sequence of SEQ ID NO: 1 of the HOXA9 gene.

11. Use of a primer set according to any one of claims 9 or 10, wherein said biological sample comprises cancer tissue chosen from the group consisting of pancreatic cancer tissue, breast cancer tissue and colon cancer tissue.

## Patentansprüche

1. Verfahren zum Bestimmen des Vorhandenseins oder Nichtvorhandenseins einer Krebszelle in einer von einem Subjekt erhaltenen biologischen Probe, umfassend die Schritte:
Extrahieren von DNA aus der biologischen Probe;
Analysieren des Methylierungsstatus für die bei dem Extraktionsschritt erhaltene DNA durch Durchführen methylierungsspezifischer PCR unter Verwendung eines Primer-Satzes, der wenigstens eine CpG-Stelle ausgewählt aus der 9., 10. und 28. bis 30. CpG-Stelle von dem 5'-Ende der Basensequenz von SEQ ID NO. 1 des HOXA9-Gens vervielfältigt; und
Bestimmen des Vorhandenseins oder Nichtvorhandenseins der Krebszelle in der biologischen Probe auf der Grundlage des Ergebnisses der PCR, wobei bestimmt wird, dass die Krebszelle in der biologischen Probe vorhanden ist, wenn das vervielfältigte Produkt der PCR nachgewiesen wird.

2. Verfahren gemäß Anspruch 1, wobei der Analyseschritt das Konzentrieren von methylierter DNA, die in der bei dem Extraktionsschritt erhaltenen DNA enthalten ist, durch Immunpräzipitation und Analysieren des Methylierungsstatus der konzentrierten methylierten DNA umfasst.

3. Verfahren gemäß einem der Ansprüche 1 bis 2, wobei das 3'-Ende eines der Primer in dem Primer-Satz Cytosin ist.

4. Verfahren gemäß einem der Ansprüche 1 bis 4, wobei der Primer-Satz die Basensequenzen SEQ ID NO: 28 und SEQ ID NO: 29 umfasst.

5. Verfahren gemäß einem der Ansprüche 1 bis 5, wobei methylierungsspezifische PCR unter Verwendung eines Primer-Satzes, der die 9., 10. und 28. bis 30. CpG-Stelle von dem 5'-Ende der Basensequenz von SEQ ID NO. 1 des HOXA9-Gens vervielfältigt.

6. Verfahren gemäß einem der Ansprüche 1 bis 5, wobei die biologische Probe Krebsgewebe ausgewählt aus der Gruppe bestehend aus Pankreaskrebsgewebe, Brustkrebsgewebe und Kolonkrebsgewebe umfasst.

7. Primer-Satz bestehend aus den Basensequenzen SEQ ID NO: 28 und SEQ ID NO: 29.

8. Kit zum Bestimmen des Vorhandenseins oder Nichtvorhandenseins einer Krebszelle in einer von einem Subjekt erhaltenen biologischen Probe, umfassend:
ein nichtmethyliertes-Cytosin-Umwandlungsmittel, das nichtmethyliertes Cytosin in aus der biologischen Probe extrahierter DNA in eine andere Base umwandelt; und
einen Primer-Satz zum Vervielfältigen der 9., 10.
und 28. bis 30. CpG-Stelle von dem 5'-Ende der Basensequenz von SEQ ID NO. 1 des HOXA9-Gens durch methylierungsspezifische PCR.

9. In-vitro-Verwendung eines Primer-Satzes, der wenigstens eine CpG-Stelle ausgewählt aus der 9., 10. und 28. bis 30. CpG-Stelle von dem 5'-Ende der Basensequenz von SEQ ID NO. 1 des HOXA9-Gens vervielfältigt, zum Bestimmen des Vorhandenseins oder Nichtvorhandenseins einer Krebszelle in einer von einem Subjekt erhaltenen biologischen Probe durch methylierungsspezifische PCR.

10. Verwendung eines Primer-Satzes gemäß Anspruch 9, der die 9., 10. und 28. bis 30. CpG-Stelle von dem 5'-Ende der Basensequenz von SEQ ID NO. 1 des HOXA9-Gens vervielfältigt.

11. Verwendung eines Primer-Satzes gemäß einem der Ansprüche 9 oder 10, wobei die biologische Probe Krebsgewebe ausgewählt aus der Gruppe bestehend aus Pankreaskrebsgewebe, Brustkrebsgewebe und Kolonkrebsgewebe umfasst.

## Revendications

1. Procédé pour la détermination de la présence ou de l'absence d'une cellule cancéreuse dans un échantillon biologique obtenu auprès d'un sujet comprenant les étapes consistant à _{:}
extraire de l'ADN à partir de l'échantillon biologique ;
analyser l'état de méthylation, pour l'ADN obtenu dans l'étape d'extraction, par la mise en oeuvre d'une PCR spécifique à la méthylation à l'aide d'un ensemble d'amorces amplifiant au moins un site CpG choisi parmi les 9^{ème}, 10^{ème} et 28^{ème} à 30^{ème} sites CpG à partir de l'extrémité 5' d'une séquence de bases de SEQ ID n° : 1 du gène HOXA9 ; et
déterminer la présence ou l'absence de la cellule cancéreuse dans l'échantillon biologique sur la base d'un résultat de la PCR,
dans lequel on détermine que la cellule cancéreuse est présente dans l'échantillon biologique lorsque le produit amplifié de la PCR est détecté.

2. Procédé selon la revendication 1, dans lequel l'étape d'analyse comprend la concentration d'ADN méthylé contenu dans l'ADN obtenu à partir de l'étape d'extraction par immunoprécipitation et l'analyse de l'état de méthylation de l'ADN méthylé concentré.

3. Procédé selon l'une quelconque des revendications 1 à 2, dans lequel l'extrémité 3' de l'une des amorces dans ledit ensemble d'amorces est la cytosine.

4. Procédé selon l'une quelconque des revendications 1 à 4, dans lequel ledit ensemble d'amorces comprend les séquences de bases SEQ ID n° : 28 et SEQ ID n° : 29.

5. Procédé selon l'une quelconque des revendications 1 à 5, dans lequel la PCR spécifique à la méthylation est effectuée à l'aide d'un ensemble d'amorces amplifiant les 9^{ème}, 10^{ème} et 28^{ème} à 30^{ème} sites CpG à partir de l'extrémité 5' d'une séquence de bases de SEQ ID n° : 1 du gène HOXA9.

6. Procédé selon l'une quelconque des revendications 1 à 5, dans lequel ledit échantillon biologique comprend du tissu cancéreux choisi dans le groupe constitué par du tissu cancéreux pancréatique, du tissu cancéreux du sein et du tissu cancéreux du côlon.

7. Ensemble d'amorces constitué des séquences de bases SEQ ID n° : 28 et SEQ ID n° : 29.

8. Kit pour la détermination de la présence ou de l'absence d'une cellule cancéreuse dans un échantillon biologique obtenu auprès d'un sujet comprenant :
un agent de conversion de cytosine non méthylée qui convertit une cytosine non méthylée dans de l'ADN extrait à partir de l'échantillon biologique en une base différente ; et
un ensemble d'amorces pour l'amplification par PCR spécifique à la méthylation des 9^{ème}, 10^{ème} et 28^{ème} à 30^{ème} sites CpG à partir de l'extrémité 5' d'une séquence de bases de SEQ ID n° : 1 du gène HOXA9.

9. Utilisation *in vitro* d'un ensemble d'amorces, amplifiant au moins un site CpG choisi parmi les 9^{ème}, 10^{ème} et 28^{ème} à 30^{ème} sites CpG à partir de l'extrémité 5' d'une séquence de bases de SEQ ID n° : 1 du HOXA9, pour la détermination de la présence ou de l'absence d'une cellule cancéreuse dans un échantillon biologique obtenu auprès d'un sujet, par PCR spécifique à la méthylation.

10. Utilisation d'un ensemble d'amorces selon la revendication 9, amplifiant les 9^{ème}, 10^{ème} et 28^{ème} à 30^{ème} sites CpG à partir de l'extrémité 5' d'une séquence de bases de SEQ ID n° : 1 du gène HOXA9.

11. Utilisation d'un ensemble d'amorces selon l'une quelconque des revendications 9 ou 10, dans laquelle ledit échantillon biologique comprend du tissu cancéreux choisi dans le groupe constitué par du tissu cancéreux pancréatique, du tissu cancéreux du sein et du tissu cancéreux du côlon.


### SEQUENCE LISTING

<110> Sysmex corporation
<120> Method for determining presence or absence of cancer cell in biological sample and molecular marker and kit for determination
<130> TM5530PC
<150> JP2010-042814
   <151> 2010-02-26
<160> 75
<170> PatentIn version 3.5
<210> 1
   <211> 548
   <212> DNA
   <213> Homo sapiens
<400> 1
   cagtcaggga caaagtgtga gtgtcaagcg tgggacagtc accccttctg gccgacagcg 60
   gttcaggttt aatgccataa ggccggctgg agggcaagcc cgcgaaggag agcgcaccgg 120
   gcgtgggctc cagccaggag cgcatgtacc tgccgtccgg cgccgccgcc gccacgggcg 180
   cctgggggtg cacgtagggg tggtggtgat ggtggtggta caccgcagcg ggtacagcgt 240
   tggcgcccgc cgcgtgcact gggttccacg aggcgccaaa caccgtcgcc ttggactgga 300
   agctgcacgg gctgaagtcg gggtgctcgg ccagcgtcgc cgcctgccgg ggaggctggc 360
   ccagggtccc cggcgcatag cggccaacgc tcagctcatc cgcggcgtcg gcgcccagca 420
   ggaacgagtc cacgtagtag ttgcccaggg ccccagtggt ggccatcacc gtgcccagcg 480
   cctggcccgc ccggcccgac ccacggaaat tatgaaactg cagatttcat gtaacaactt 540
   ggtggcac 548
<210> 2
   <211> 577
   <212> DNA
   <213> Homo sapiens
<400> 2
   cagaatgaac tcctcggcat cgctttgaat ccgggctcac atatggttgc taattagctg 60
   gctatctctt ggagttaaat gatttttttt ctcgccccgt tcgcatccat aatagacgcg 120
   aacagagtcg tgattgttcc ctgttgcggg acattctgcg cctgaatgag cgggggctgc 180
   tgtgtcgacg cggatttgcc atatggttgc gcggagccag gaagaatggg cccctgtgtg 240
   tgcgaccggg acgcagcggc gcgggccgtg ccagccgggc ggagccgggt gcccgacggc 300
   gcaggacgag cgggcgcagg acgagcgagc gcagcgggcg cgggcgcacc gtcggggcgc 360
   ggggaccccg agggccagcg gagccgtgct gcgcgcaggg atggacgctg ggcgcccaag 420
   gcccgttccc gtggacaggc gcgcggaggg cagcaggacc gatgcaccga cggacgcacc 480
   gacggacgcg cggacagatg cgccgaccgc aggctctccc gccgtcccgg ggccgtcgtg 540
   cccgtgcagg ccgcgagcag cggcggggcc ggctctg 577
<210> 3
   <211> 334
   <212> DNA
   <213> Homo sapiens
<400> 3
   tcgggtggga gccggaatgg ggcctggatg gagagcaggc gccaccctgg gaacaaagct 60
   ggttcagccc gtctgcagct gtcgctgcca gttggggcca catggcctct gaggaatgtc 120
   agcagcaagg tcacgtgggc ctctgctgca cccacccgcg agctgggacg tggcttccct 180
   gagcatacag gcaccttgcc acctgggcac ccccttgggc gtcccttagg cacctctgac 240
   actgaccctg accgccctgg cagcacgggc cctcccagca gccttgccaa ggggccagct 300
   ccagcaagtg ggcaggcctt gggctagagg tgct 334
<210> 4
   <211> 38
   <212> DNA
   <213> Homo sapiens
<400> 4
   catgcccaac ctgctgggcc acacgtcgca gggcgagg 38
<210> 5
   <211> 40
   <212> DNA
   <213> Homo sapiens
<400> 5
   accaggtctc gacgcccatt cccgcctgcc ggcccatgtg 40
<210> 6
   <211> 55
   <212> DNA
   <213> Homo sapiens
<400> 6
   gatcaccagg cagcacctgg acagctccag agtcggggaa gcgccatggt tcctg 55
<210> 7
   <211> 567
   <212> DNA
   <213> Homo sapiens
<400> 7
   ggaagaggcc caagtggacc tccttcctcc accctggaac tctggccaga ggcagagcgc 60
   gaggtgagga gtccagggcc ccagagccca ggattggcgg cggctttgtc ttcaatcgtc 120
   cacctgtatc tcctcgtctg acaggtgggg ccgggagtca gggccggcag ggccgaggca 180
   gaggccggga tctggagcgc cttcgggcag tgctaagctg cacaggactt ccggggacaa 240
   cgcgcgtagc gaggcgacgt cggcgcgcat ctcctccaca tcgcgcttga gcttggctcg 300
   ccggttctgg aaccaagtga ccacctgcgc gttggccagg ccgagtcgcg tagctagccc 360
   gtctcgctcg gacggcgcca ggtacttctg gaagacgaag cgccgctcca gctccagcac 420
   ctgttgcgcg gtgaacgcag tgcgtgactt gcgccgtttg cggccgaagg gaccagggcc 480
   cagcgcgtcc ggacctgccc gccctgtagg gatagggagg gggtcagttt ccagcctccg 540
   ggaatcaggc tgggcctgga aagaaaa 567
<210> 8
   <211> 692
   <212> DNA
   <213> Homo sapiens
<400> 8
   agctaggact gtgcaacacc agtctccgct ctcggagtcg gcacgggggc tggtgggtgg 60
   cgggatgaac tcggggaagg cgacaggctg gaaacgcagc tcctcgactt tgcagcgcac 120
   caacccccta tcgccccgtc cgctgctcag gtgcgaggat tcgccagacg ccacaggact 180
   gggggcgact cgcaagggca tcatgccacc tagaggaaac gcgcagtgag tgagccctgg 240
   gtcactgaaa atgggagaac gttcgagtcg gtcaagatga ctccggcctg cgagttgatt 300
   cagactcctc ggaagccaga aattgggtct agagtgggtt ggtccctccg tcagcagttc 360
   gggcccagcc gaagcttggg gcctagaggg cagacgtccg gctctgatca gagtacagcg 420
   ggctgggcct ggcagcctgc aggcaacctg gaccttgcaa taaagctgca ttcaacataa 480
   ctcagaccac agtgggagcc cccgaagggc ttcatgtgag tcccgaaacc agagtttaga 540
   ggcgcggcct ggttctcgtt tatcttttcc agacagcatt taggccaata aggtctttga 600
   aaggaggcag ccccaaagcc ctcaggccga cacctgaggg cagggtctca aacagctgtg 660
   cttgaagaac cggcaatctt gcttttccca ca 692
<210> 9
   <211> 586
   <212> DNA
   <213> Homo sapiens
<400> 9
   gcacattttt agggaggagg ggggggatga agatgaggcg gatggcgtga ggtatttgag 60
   ggggacgagt ctgcagatga ggaaaggaaa tgaagtggat tgtcgccagt gccttggaaa 120
   cgaaggggga taagggtggg gaacgaagtt tgttgtgtgt gttagggtgg ggttggccca 180
   agggaccatg aatcattaga atggcgctcg gaggggtgtg cttgcaagtg tactggtgca 240
   ggagggacgt ggaagtcgtg ggtggcagag aaaagtgtgc accgctggga ggtctatttc 300
   aagaataggt tgggatgaga aacatgtctg ggcgaggggg gtgggaggcc cggtgtcacc 360
   tgtggatgga tccacagccc tgcaattgcg tgttgcaaag tttgcccggc cccgcattca 420
   ttttgaaggt tcttggcgat gcctcgactt ttcgcctcaa agaagtgagc tctcataagg 480
   ttcagaggcc gggagccaaa gcgggagtga agactctcga ggggtccaaa tgaagcagaa 540
   gcgagagccc ctgtcctccc cctatgtgga ggtgaagcag gggttg 586
<210> 10
   <211> 508
   <212> DNA
   <213> Homo sapiens
<400> 10
   gtggtggtgg ggtcagtgga gaggaggggt tgttcagaga gggctctcta aagggaagag 60
   aggtggaaaa tatgtgtgcg ggtggagtgg gggttggggg tggggttaca agcaatttgg 120
   gagggaacag aatccaggtg aaatatggga gtgatggaat acaggctgaa agtgggatgt 180
   gtggagcaga accttgagca aatatgcggg aagaatgcag ttggaataac ggggtgcatt 240
   gaagttgaat atgcgggagc cctaggagac cgtgggtggc tggtgtaggg aggagagtgc 300
   ggctgcctcc ctgcccccaa atctgggaga gcaggatgga atgaaggggc tttggacggg 360
   agagaggggg ctggtttccg gggaatgtag catgagggtt gggctggaat atgtggagtg 420
   ggaggttctg cagagaggag gggaagggga gtccagaatt ccgtggccat ctgggggaca 480
   agtgacgggc agctggggtc agggaggc 508
<210> 11
   <211> 508
   <212> DNA
   <213> Homo sapiens
<400> 11
   gcctgggtag cccattggcc cgcggccggg tccgacgtca gtgatgacag gtcctgccgc 60
   acgcggtcgg tctctctctc tctctctcac acttccctgc tccaaccgcc accccctcac 120
   acacctctcc cccaaacata taccctcttt ccctccctca ccccaccccc acagcatctc 180
   aagcttccac tgctcatgcg ctctgacccg ggcacaaatc cttttctctc cccttgagcc 240
   tcctccccct cctctccgga aagctgagtg gatcagcttt gactcagggc tcctggaagt 300
   actcggggct gggttatgcc accattcacc acaactttgg ggaggcagca ttgcgggacc 360
   tcagtcgggg ctgatgggca gcatcgtgtg cgcagagatg gcggtctcgt tgggcagttt 420
   gtccctgcag cctgcaagag gagggggtcg ggatcgaagg accgaaggag gttgctgact 480
   caggagccag gagctgagaa actcctag 508
<210> 12
   <211> 655
   <212> DNA
   <213> Homo sapiens
<400> 12
   gaggtgacac taaggactcc tgggtgctaa aagagggagg ggctggggtc tgaactgtgg 60
   ttgttgctgc tataggagtc gagactgaga ccaccggtca gggaatggga gccagcccag 120
   gggctcggcg tgcaccggcc atggcggggg ctgtgcagga ggcggtgccg agcgagagcc 180
   cggcttactt gggatagaac cgaaagtccc gcgccgaggt ggtcagatag agctggcggc 240
   ggttcaggac gccccggtcg gatactgtga agggtgggcc agacagcgcg gggttcttcg 300
   tgcaggcgat tagagcctga gcaagattgg gggaggaggc atacccaaga catagatttc 360
   ataccccatc ccaagtcctg ggtcccgagt tcagaatccc tctccttcgg cctcgacagc 420
   catgacggcc acggccctca caccccgccc cagggtcaga gcccttcctg ccacccacgc 480
   cagggaactt cagcttcctt tctcataccc gaacccttcc cagcctcacc tccctaaatc 540
   caagccctcc tgctggtcaa ggaactcaaa tgcaggcccc cagccaccac aaaatccccc 600
   tggttctgcc cccggtctca agccttcttg gtgtggccac tggagctgag ctgca 655
<210> 13
   <211> 530
   <212> DNA
   <213> Homo sapiens
<400> 13
   taacacaata cttgagtgta cagacaaaac agattttaga ctaaaagaac tatgtcacca      60
   gcaaataatg aaacttcatc acacaaactc ctatgcgctt gccaaagttc cccgtacctt     120
   tcaacttgcc cgtagctgaa gggctccctg tacaatggaa gcccttctgc cttgctcaag     180
   actggatgtc cctcttttct aacttgccta aacaccatcg gcacacagca cagtcctttg     240
   agtgggtctc acactggaca cagcatcgag gagcttctcc acggggaagt gtgtgtgtga     300
   gagcctgcct gtgtgtcaac actgtccaag actcggagct gcgactcggt cgcgtggtgc     360
   ggagtccagg gctgagaagg cagcgcacta aatatctctg tatcaggtaa aaaagccgtt     420
   tggtgatgac tcgccaccag gcaaatgttg ggagtcccaa cgatttttcc cgtggaatgc     480
   accgagggtc gccatggatg tgcctgcagg ggctgtgagt atcaggcaga                530
<210> 14
   <211> 436
   <212> DNA
   <213> Homo sapiens
<400> 14
   gcagcagcag agtctgcaga ggggtgggag ggaagcgcag cccacccggg gcttcaggat      60
   aaagaccagg cgtggggaac cgcctctgcc cttctaaacc ctccagtttg gtctccactg     120
   ttccaaggcc ccgtcccacc ccgagcagta ctccccgcta aagccacgcc ccgagcggcc     180
   aaaccccgcc cacttccgcc cacctacccc gaaagcccca cccattctca agctccgccc     240
   cctgagcacc tgcctgctct ttcccagtcc cgcccgtcct caccttcagc tgcaattttc     300
   tcgaggcgga gatcttagat tttttctgcc agggtgagat ggagcaagga aggatcatgg     360
   agggggattc ggagacgacg gtggagggga cctcaagaca cccccagcaa acccagccgg     420
   tccagatttg ggccca                                                     436
<210> 15
   <211> 579
   <212> DNA
   <213> Homo sapiens
<400> 15
   cagtcaggga caaagtgtga gtgtcaagcg tgggacagtc accccttctg gccgacagcg 60
   gttcaggttt aatgccataa ggccggctgg agggcaagcc cgcgaaggag agcgcaccgg 120
   gcgtgggctc cagccaggag cgcatgtacc tgccgtccgg cgccgccgcc gccacgggcg 180
   cctgggggtg cacgtagggg tggtggtgat ggtggtggta caccgcagcg ggtacagcgt 240
   tggcgcccgc cgcgtgcact gggttccacg aggcgccaaa caccgtcgcc ttggactgga 300
   agctgcacgg gctgaagtcg gggtgctcgg ccagcgtcgc cgcctgccgg ggaggctggc 360
   ccagggtccc cggcgcatag cggccaacgc tcagctcatc cgcggcgtcg gcgcccagca 420
   ggaacgagtc cacgtagtag ttgcccaggg ccccagtggt ggccatcacc gtgcccagcg 480
   cctggcccgc ccggcccgac ccacggaaat tatgaaactg cagatttcat gtaacaactt 540
   ggtggcaccg ggggggaagt acagtcacct aataagttg 579
<210> 16
   <211> 642
   <212> DNA
   <213> Homo sapiens
<400> 16
   gggacatctg tcttctatac ctaataaagt cgcccgacgc agaatgaact cctcggcatc 60
   gctttgaatc cgggctcaca tatggttgct aattagctgg ctatctcttg gagttaaatg 120
   attttttttc tcgccccgtt cgcatccata atagacgcga acagagtcgt gattgttccc 180
   tgttgcggga cattctgcgc ctgaatgagc gggggctgct gtgtcgacgc ggatttgcca 240
   tatggttgcg cggagccagg aagaatgggc ccctgtgtgt gcgaccggga cgcagcggcg 300
   cgggccgtgc cagccgggcg gagccgggtg cccgacggcg caggacgagc gggcgcagga 360
   cgagcgagcg cagcgggcgc gggcgcaccg tcggggcgcg gggaccccga gggccagcgg 420
   agccgtgctg cgcgcaggga tggacgctgg gcgcccaagg cccgttcccg tggacaggcg 480
   cgcggagggc agcaggaccg atgcaccgac ggacgcaccg acggacgcgc ggacagatgc 540
   gccgaccgca ggctctcccg ccgtcccggg gccgtcgtgc ccgtgcaggc cgcgagcagc 600
   ggcggggccg gctctgcggc tgcagcagcc ccattgtgag gc 642
<210> 17
   <211> 673
   <212> DNA
   <213> Homo sapiens
<400> 17
   aggcactcat ggggcaggaa tcacagtgcc agctgccccc cgaaacgtgg gaacaaaagc 60
   agcgtcgggt gggagccgga atggggcctg gatggagagc aggcgccacc ctgggaacaa 120
   agctggttca gcccgtctgc agctgtcgct gccagttggg gccacatggc ctctgaggaa 180
   tgtcagcagc aaggtcacgt gggcctctgc tgcacccacc cgcgagctgg gacgtggctt 240
   ccctgagcat acaggcacct tgccacctgg gcaccccctt gggcgtccct taggcacctc 300
   tgacactgac cctgaccgcc ctggcagcac gggccctccc agcagccttg ccaaggggcc 360
   agctccagca agtgggcagg ccttgggcta gaggtgctcg ggggaaggag agcgaggctg 420
   gacctgcagg tccggggaag ccaggtgagc gcccagccct gcttcttccc catcaagaca 480
   cccctgaccg cagcatcccc tggctctgtt cctgaaattc actgagcacc gtgggtcact 540
   catccaggcc caggctgaca ccattcacca actctcacct tagagagtct gggaaagacc 600
   tccagctggt gagaggcctg agctctagcc tggcttcctt cgaactctca gtgggaccct 660
   agccaagtga cct 673
<210> 18
   <211> 492
   <212> DNA
   <213> Homo sapiens
<400> 18
   gctgctgctg tggcagctgc tggcggcggg cggcgcggca ctggagatcg gccgcttcga 60
   cccggagcgc gggcgcgggg ctgcgccgtg ccaggcggtg gagatcccca tgtgccgcgg 120
   catcggctac aacctgaccc gcatgcccaa cctgctgggc cacacgtcgc agggcgaggc 180
   ggctgccgag ctagcggagt tcgcgccgct ggtgcagtac ggctgccaca gccacctgcg 240
   cttcttcctg tgctcgctct acgcgcccat gtgcaccgac caggtctcga cgcccattcc 300
   cgcctgccgg cccatgtgcg agcaggcgcg cctgcgctgc gcgcccatca tggagcagtt 360
   caacttcggc tggccggact cgctcgactg cgcccggctg cccacgcgca acgacccgca 420
   cgcgctgtgc atggaggcgc ccgagaacgc cacggccggc cccgcggagc cccacaaggg 480
   cctgggcatg ct 492
<210> 19
   <211> 654
   <212> DNA
   <213> Homo sapiens
<400> 19
   tggagtgggg ccccacttta ccttgcccac atacaggggc tccgtgcccg tgtactcctc 60
   taccacgaag aactggttcc acacccagcc gcgtttgacg cggccggctc ccagcgcgcc 120
   gtcctgccga gctccgggcg ccgacggcga gggtgtgccc gctgcccaca ggcgccccag 180
   cagcgtcggc ggcggcggca gcagcagcag cagcagtagc gcgggggaca gcgcgactcc 240
   cgcccggagc cccctacctt cgggcctcgg cctcatcctt ggcctgcgcg gggctggggc 300
   ccaggagcat ggacgagagg caccagggcg ccgctgcttg gtcgcacaac gatgcggcgc 360
   cgtgtcacat ggtggcctca gcgcggccgc cgggatgtcg cccccgacgg ggcacccgga 420
   cgggcccgcg gccctgaacg ccgcccagcc gcgggggtac ccggctggag ggggaggggg 480
   cgcggccgca tccggggcat ggacagcccc cggggtgccc gcccgcgccc ccgtcgccgc 540
   gtcgcgtgcg gatcaccagg cagcacctgg acagctccag agtcggggaa gcgccatggt 600
   tcctgcgcag aaaggatgcg ggttggggcc ggcagatcct gccaggacta gggg 654
<210> 20
   <211> 567
   <212> DNA
   <213> Homo sapiens
<400> 20
   ggaagaggcc caagtggacc tccttcctcc accctggaac tctggccaga ggcagagcgc 60
   gaggtgagga gtccagggcc ccagagccca ggattggcgg cggctttgtc ttcaatcgtc 120
   cacctgtatc tcctcgtctg acaggtgggg ccgggagtca gggccggcag ggccgaggca 180
   gaggccggga tctggagcgc cttcgggcag tgctaagctg cacaggactt ccggggacaa 240
   cgcgcgtagc gaggcgacgt cggcgcgcat ctcctccaca tcgcgcttga gcttggctcg 300
   ccggttctgg aaccaagtga ccacctgcgc gttggccagg ccgagtcgcg tagctagccc 360
   gtctcgctcg gacggcgcca ggtacttctg gaagacgaag cgccgctcca gctccagcac 420
   ctgttgcgcg gtgaacgcag tgcgtgactt gcgccgtttg cggccgaagg gaccagggcc 480
   cagcgcgtcc ggacctgccc gccctgtagg gatagggagg gggtcagttt ccagcctccg 540
   ggaatcaggc tgggcctgga aagaaaa 567
<210> 21
   <211> 738
   <212> DNA
   <213> Homo sapiens
<400> 21
   agctaggact gtgcaacacc agtctccgct ctcggagtcg gcacgggggc tggtgggtgg 60
   cgggatgaac tcggggaagg cgacaggctg gaaacgcagc tcctcgactt tgcagcgcac 120
   caacccccta tcgccccgtc cgctgctcag gtgcgaggat tcgccagacg ccacaggact 180
   gggggcgact cgcaagggca tcatgccacc tagaggaaac gcgcagtgag tgagccctgg 240
   gtcactgaaa atgggagaac gttcgagtcg gtcaagatga ctccggcctg cgagttgatt 300
   cagactcctc ggaagccaga aattgggtct agagtgggtt ggtccctccg tcagcagttc 360
   gggcccagcc gaagcttggg gcctagaggg cagacgtccg gctctgatca gagtacagcg 420
   ggctgggcct ggcagcctgc aggcaacctg gaccttgcaa taaagctgca ttcaacataa 480
   ctcagaccac agtgggagcc cccgaagggc ttcatgtgag tcccgaaacc agagtttaga 540
   ggcgcggcct ggttctcgtt tatcttttcc agacagcatt taggccaata aggtctttga 600
   aaggaggcag ccccaaagcc ctcaggccga cacctgaggg cagggtctca aacagctgtg 660
   cttgaagaac cggcaatctt gcttttccca cacgcagctg cacctctcct gagcgctgca 720
   gctgatggct gtatagtg 738
<210> 22
   <211> 586
   <212> DNA
   <213> Homo sapiens
<400> 22
   gcacattttt agggaggagg ggggggatga agatgaggcg gatggcgtga ggtatttgag 60
   ggggacgagt ctgcagatga ggaaaggaaa tgaagtggat tgtcgccagt gccttggaaa 120
   cgaaggggga taagggtggg gaacgaagtt tgttgtgtgt gttagggtgg ggttggccca 180
   agggaccatg aatcattaga atggcgctcg gaggggtgtg cttgcaagtg tactggtgca 240
   ggagggacgt ggaagtcgtg ggtggcagag aaaagtgtgc accgctggga ggtctatttc 300
   aagaataggt tgggatgaga aacatgtctg ggcgaggggg gtgggaggcc cggtgtcacc 360
   tgtggatgga tccacagccc tgcaattgcg tgttgcaaag tttgcccggc cccgcattca 420
   ttttgaaggt tcttggcgat gcctcgactt ttcgcctcaa agaagtgagc tctcataagg 480
   ttcagaggcc gggagccaaa gcgggagtga agactctcga ggggtccaaa tgaagcagaa 540
   gcgagagccc ctgtcctccc cctatgtgga ggtgaagcag gggttg 586
<210> 23
   <211> 657
   <212> DNA
   <213> Homo sapiens
<400> 23
   ccaccaggat gagccactgg tcattccgaa gcaaggatgg aggatgcagg tagagggaca 60
   gggtcagcgg cgggagggga gagggggctc agggtccggg gaatcctaga agtggcggtg 120
   gtggtggggt cagtggagag gaggggttgt tcagagaggg ctctctaaag ggaagagagg 180
   tggaaaatat gtgtgcgggt ggagtggggg ttgggggtgg ggttacaagc aatttgggag 240
   ggaacagaat ccaggtgaaa tatgggagtg atggaataca ggctgaaagt gggatgtgtg 300
   gagcagaacc ttgagcaaat atgcgggaag aatgcagttg gaataacggg gtgcattgaa 360
   gttgaatatg cgggagccct aggagaccgt gggtggctgg tgtagggagg agagtgcggc 420
   tgcctccctg cccccaaatc tgggagagca ggatggaatg aaggggcttt ggacgggaga 480
   gagggggctg gtttccgggg aatgtagcat gagggttggg ctggaatatg tggagtggga 540
   ggttctgcag agaggagggg aaggggagtc cagaattccg tggccatctg ggggacaagt 600
   gacgggcagc tggggtcagg gaggccgtgt ggagctggca cgggtgtggg atttatt 657
<210> 24
   <211> 508
   <212> DNA
   <213> Homo sapiens
<400> 24
   gcctgggtag cccattggcc cgcggccggg tccgacgtca gtgatgacag gtcctgccgc 60
   acgcggtcgg tctctctctc tctctctcac acttccctgc tccaaccgcc accccctcac 120
   acacctctcc cccaaacata taccctcttt ccctccctca ccccaccccc acagcatctc 180
   aagcttccac tgctcatgcg ctctgacccg ggcacaaatc cttttctctc cccttgagcc 240
   tcctccccct cctctccgga aagctgagtg gatcagcttt gactcagggc tcctggaagt 300
   actcggggct gggttatgcc accattcacc acaactttgg ggaggcagca ttgcgggacc 360
   tcagtcgggg ctgatgggca gcatcgtgtg cgcagagatg gcggtctcgt tgggcagttt 420
   gtccctgcag cctgcaagag gagggggtcg ggatcgaagg accgaaggag gttgctgact 480
   caggagccag gagctgagaa actcctag 508
<210> 25
   <211> 655
   <212> DNA
   <213> Homo sapiens
<400> 25
   gaggtgacac taaggactcc tgggtgctaa aagagggagg ggctggggtc tgaactgtgg 60
   ttgttgctgc tataggagtc gagactgaga ccaccggtca gggaatggga gccagcccag 120
   gggctcggcg tgcaccggcc atggcggggg ctgtgcagga ggcggtgccg agcgagagcc 180
   cggcttactt gggatagaac cgaaagtccc gcgccgaggt ggtcagatag agctggcggc 240
   ggttcaggac gccccggtcg gatactgtga agggtgggcc agacagcgcg gggttcttcg 300
   tgcaggcgat tagagcctga gcaagattgg gggaggaggc atacccaaga catagatttc 360
   ataccccatc ccaagtcctg ggtcccgagt tcagaatccc tctccttcgg cctcgacagc 420
   catgacggcc acggccctca caccccgccc cagggtcaga gcccttcctg ccacccacgc 480
   cagggaactt cagcttcctt tctcataccc gaacccttcc cagcctcacc tccctaaatc 540
   caagccctcc tgctggtcaa ggaactcaaa tgcaggcccc cagccaccac aaaatccccc 600
   tggttctgcc cccggtctca agccttcttg gtgtggccac tggagctgag ctgca 655
<210> 26
   <211> 680
   <212> DNA
   <213> Homo sapiens
<400> 26
   aataagattt gtccagtctc attgccaaaa tagttctgag cttttggttc cggtaactac 60
   agtggaataa gtctttactc tccaagatag ggctgcaact ttattatttt ttattagcaa 120
   tagtattggt gtcataagca aaataaaacg taacacaata cttgagtgta cagacaaaac 180
   agattttaga ctaaaagaac tatgtcacca gcaaataatg aaacttcatc acacaaactc 240
   ctatgcgctt gccaaagttc cccgtacctt tcaacttgcc cgtagctgaa gggctccctg 300
   tacaatggaa gcccttctgc cttgctcaag actggatgtc cctcttttct aacttgccta 360
   aacaccatcg gcacacagca cagtcctttg agtgggtctc acactggaca cagcatcgag 420
   gagcttctcc acggggaagt gtgtgtgtga gagcctgcct gtgtgtcaac actgtccaag 480
   actcggagct gcgactcggt cgcgtggtgc ggagtccagg gctgagaagg cagcgcacta 540
   aatatctctg tatcaggtaa aaaagccgtt tggtgatgac tcgccaccag gcaaatgttg 600
   ggagtcccaa cgatttttcc cgtggaatgc accgagggtc gccatggatg tgcctgcagg 660
   ggctgtgagt atcaggcaga 680
<210> 27
   <211> 527
   <212> DNA
   <213> Homo sapiens
<400> 27
   gcagcagcag agtctgcaga ggggtgggag ggaagcgcag cccacccggg gcttcaggat 60
   aaagaccagg cgtggggaac cgcctctgcc cttctaaacc ctccagtttg gtctccactg 120
   ttccaaggcc ccgtcccacc ccgagcagta ctccccgcta aagccacgcc ccgagcggcc 180
   aaaccccgcc cacttccgcc cacctacccc gaaagcccca cccattctca agctccgccc 240
   cctgagcacc tgcctgctct ttcccagtcc cgcccgtcct caccttcagc tgcaattttc 300
   tcgaggcgga gatcttagat tttttctgcc agggtgagat ggagcaagga aggatcatgg 360
   agggggattc ggagacgacg gtggagggga cctcaagaca cccccagcaa acccagccgg 420
   tccagatttg ggcccacgtc caacttgagc cctgagtcta cgggaggcca cgcccctcat 480
   tcccatccac caatctgggt ctcttccttt ggataggcac ttcccat 527
<210> 28
   <211> 20
   <212> DNA
   <213> Artificial Sequence
<220>
   <223> primer
<400> 28
   cgtgggtttt agttaggagc 20
<210> 29
   <211> 23
   <212> DNA
   <213> Artificial Sequence
<220>
   <223> primer
<400> 29
   atccaaaacg acgatattta acg 23
<210> 30
   <211> 26
   <212> DNA
   <213> Artificial Sequence
<220>
   <223> primer
<400> 30
   cgtttcgttc gtatttataa tagacg 26
<210> 31
   <211> 23
   <212> DNA
   <213> Artificial Sequence
<220>
   <223> primer
<400> 31
   aaaacccatt cttcctaact ccg 23
<210> 32
   <211> 27
   <212> DNA
   <213> Artificial Sequence
<220>
   <223> primer
<400> 32
   tgaggaatgt tagtagtaag gttacgt 27
<210> 33
   <211> 25
   <212> DNA
   <213> Artificial Sequence
<220>
   <223> primer
<400> 33
   cctatatact caaaaaaacc acgtc 25
<210> 34
   <211> 22
   <212> DNA
   <213> Artificial Sequence
<220>
   <223> primer
<400> 34
   gttgggttat acgtcgtagg gc 22
<210> 35
   <211> 23
   <212> DNA
   <213> Artificial Sequence
<220>
   <223> primer
<400> 35
   gacaaacgaa aataaacgtc gaa 23
<210> 36
   <211> 23
   <212> DNA
   <213> Artificial Sequence
<220>
   <223> primer
<400> 36
   gatagtttta gagtcgggga agc 23
<210> 37
   <211> 25
   <212> DNA
   <213> Artificial Sequence
<220>
   <223> primer
<400> 37
   cctaatccta acaaaatcta ccgac 25
<210> 38
   <211> 23
   <212> DNA
   <213> Artificial Sequence
<220>
   <223> primer
<400> 38
   ttttagagtt taggattggc ggc 23
<210> 39
   <211> 25
   <212> DNA
   <213> Artificial Sequence
<220>
   <223> primer
<400> 39
   tacaacttaa cactacccga aaacg 25
<210> 40
   <211> 24
   <212> DNA
   <213> Artificial Sequence
<220>
   <223> primer
<400> 40
   ataggttgga aacgtagttt ttcg 24
<210> 41
   <211> 24
   <212> DNA
   <213> Artificial Sequence
<220>
   <223> primer
<400> 41
   ctataacgtc taacgaatcc tcgc 24
<210> 42
   <211> 25
   <212> DNA
   <213> Artificial Sequence
<220>
   <223> primer
<400> 42
   tgttgtaaag tttgttcggt ttcgt 25
<210> 43
   <211> 25
   <212> DNA
   <213> Artificial Sequence
<220>
   <223> primer
<400> 43
   ttctacttca tttaaacccc tcgaa 25
<210> 44
   <211> 30
   <212> DNA
   <213> Artificial Sequence
<220>
   <223> primer
<400> 44
   tgtgtggagt agaattttga gtaaatatgc 30
<210> 45
   <211> 26
   <212> DNA
   <213> Artificial Sequence
<220>
   <223> primer
<400> 45
   aatttaaaaa caaaaaaaca accgca 26
<210> 46
   <211> 21
   <212> DNA
   <213> Artificial Sequence
<220>
   <223> primer
<400> 46
   gtagtttatt ggttcgcggt c 21
<210> 47
   <211> 27
   <212> DNA
   <213> Artificial Sequence
<220>
   <223> primer
<400> 47
   aaaaaaaata tataaaaaaa taacgat 27
<210> 48
   <211> 27
   <212> DNA
   <213> Artificial Sequence
<220>
   <223> primer
<400> 48
   agagttcggt ttatttggga tagaatc 27
<210> 49
   <211> 20
   <212> DNA
   <213> Artificial Sequence
<220>
   <223> primer
<400> 49
   gaccgaaacg tcctaaaccg 20
<210> 50
   <211> 25
   <212> DNA
   <213> Artificial Sequence
<220>
   <223> primer
<400> 50
   tattgtttaa gattcggagt tgcga 25
<210> 51
   <211> 25
   <212> DNA
   <213> Artificial Sequence
<220>
   <223> primer
<400> 51
   ctcccaacat ttacctaata acgaa 25
<210> 52
   <211> 22
   <212> DNA
   <213> Artificial Sequence
<220>
   <223> primer
<400> 52
   gggagggaag cgtagtttat tc 22
<210> 53
   <211> 28
   <212> DNA
   <213> Artificial Sequence
<220>
   <223> primer
<400> 53
   ctaaaaaatt taaaaaaaca aaaacgat 28
<210> 54
   <211> 26
   <212> DNA
   <213> Artificial Sequence
<220>
   <223> primer
<400> 54
   tagttaggga taaagtgtga gtgtta 26
<210> 55
   <211> 27
   <212> DNA
   <213> Artificial Sequence
<220>
   <223> primer
<400> 55
   caacttatta aataactata cttcccc 27
<210> 56
   <211> 30
   <212> DNA
   <213> Artificial Sequence
<220>
   <223> primer
<400> 56
   gggatatttg ttttttatat ttaataaagt 30
<210> 57
   <211> 25
   <212> DNA
   <213> Artificial Sequence
<220>
   <223> primer
<400> 57
   acctcacaat aaaactacta caacc 25
<210> 58
   <211> 25
   <212> DNA
   <213> Artificial Sequence
<220>
   <223> primer
<400> 58
   aggtatttat ggggtaggaa ttata 25
<210> 59
   <211> 25
   <212> DNA
   <213> Artificial Sequence
<220>
   <223> primer
<400> 59
   aaatcactta actaaaatcc cacta 25
<210> 60
   <211> 25
   <212> DNA
   <213> Artificial Sequence
<220>
   <223> primer
<400> 60
   ggaagaggtt taagtggatt ttttt 25
<210> 61
   <211> 22
   <212> DNA
   <213> Artificial Sequence
<220>
   <223> primer
<400> 61
   ttttctttcc aaacccaacc ta 22
<210> 62
   <211> 26
   <212> DNA
   <213> Artificial Sequence
<220>
   <223> primer
<400> 62
   agttaggatt gtgtaatatt agtttt 26
<210> 63
   <211> 24
   <212> DNA
   <213> Artificial Sequence
<220>
   <223> primer
<400> 63
   cactatacaa ccatcaacta caac 24
<210> 64
   <211> 24
   <212> DNA
   <213> Artificial Sequence
<220>
   <223> primer
<400> 64 24
   gtatattttt agggaggagg gggg 24
<210> 65
   <211> 24
   <212> DNA
   <213> Artificial Sequence
<220>
   <223> primer
<400> 65
   caacccctac ttcacctcca cata 24
<210> 66
   <211> 26
   <212> DNA
   <213> Artificial Sequence
<220>
   <223> primer
<400> 66
   ttattaggat gagttattgg ttattt 26
<210> 67
   <211> 20
   <212> DNA
   <213> Artificial Sequence
<220>
   <223> primer
<400> 67
   acctccctaa ccccaactac 20
<210> 68
   <211> 20
   <212> DNA
   <213> Artificial Sequence
<220>
   <223> primer
<400> 68
   gtttgggtag tttattggtt 20
<210> 69
   <211> 25
   <212> DNA
   <213> Artificial Sequence
<220>
   <223> primer
<400> 69
   ctaaaaattt ctcaactcct aactc 25
<210> 70
   <211> 25
   <212> DNA
   <213> Artificial Sequence
<220>
   <223> primer
<400> 70
   gaggtgatat taaggatttt tgggt 25
<210> 71
   <211> 25
   <212> DNA
   <213> Artificial Sequence
<220>
   <223> primer
<400> 71
   tacaactcaa ctccaataac cacac 25
<210> 72
   <211> 30
   <212> DNA
   <213> Artificial Sequence
<220>
   <223> primer
<400> 72
   aataagattt gtttagtttt attgttaaaa 30
<210> 73
   <211> 26
   <212> DNA
   <213> Artificial Sequence
<220>
   <223> primer
<400> 73
   tctacctaat actcacaacc cctaca 26
<210> 74
   <211> 26
   <212> DNA
   <213> Artificial Sequence
<220>
   <223> primer
<400> 74
   gtagtagtag agtttgtaga ggggtg 26
<210> 75
   <211> 25
   <212> DNA
   <213> Artificial Sequence
<220>
   <223> primer
<400> 75
   ataaaaaata cctatccaaa aaaaa 25